# EUROPÄISCHE PATENTSCHRIFT

(11) **EP 3 502 275 B1**
(45) Veröffentlichungstag und Bekanntmachung des Hinweises auf die Patenterteilung: **07.06.2023**
(21) Anmeldenummer: 18192256.8
(22) Anmeldetag: 03.09.2018
(51) Int. Cl.: C12Q 1/6806, C12N 11/08, C12M 1/00, G01N 33/569, C12N 15/10

(54) **VERFAHREN ZUR ANREICHERUNG VON NUKLEINSÄUREN**
METHOD FOR ENRICHMENT OF NUCLEIC ACIDS
PROCÉDÉ D'ENRICHISSEMENT DES ACIDES NUCLÉIQUES

(30) Priorität: 22.12.2017 EP 17210121
(43) Veröffentlichungstag der Anmeldung: 26.06.2019
(73) Patentinhaber: Attomol GmbH Molekulare Diagnostika, 03205 Bronkow (DE)
(72) Erfinder: Lehmann, Werner, 03205 Bronkow (DE); Milius, Stephan, 02991 Lauta Dorf (DE); Berger, Enrico, 01968 Senftenberg Niemtsch (DE)
(74) Vertreter: Gulde & Partner

(56) Entgegenhaltungen:
- WO-A1-01/94572
- WO-A1-2004/055207
- WO-A1-2014/186607
- WO-A2-99/29703
- DE-A1- 2 552 510
- DE-A1- 10 236 101
- US-A1- 2005 009 036
- M. Correia: "UV light effect on proteins and new approaches in nanomedicine", , 9. März 2015 (2015-03-09), XP055446694, Gefunden im Internet: URL:http://www.hst.aau.dk/digitalAssets/10 1/101816_manuel_correia_phd.pdf [gefunden am 2018-02-01]

## Beschreibung

Die Erfindung betrifft ein Verfahren zur Anreicherung von Nukleinsäuren.

Die Analyse von Zellen, die physiologisch oder pathophysiologisch in Körperflüssigkeiten vorkommen, setzt meist vor der Analyse die Anreicherung oder Reinigung dieser Zellen oder ihrer Bestandteile voraus. Die wichtigsten Probenmatrices für Analysen sind Blut, Abstrichmaterialien, Liquor und Urin welche häufig - z.B. im Rahmen der medizinischen Diagnostik - auf zytologische, immunologische oder molekularbiologische Marker oder auf Erreger von Infektionserkrankungen untersucht werden müssen. Es wurden vielfältige Verfahren entwickelt, um gezielt einzelne Populationen der Blutzellen wie z.B. Erythrozyten oder Leukozyten oder Nukleinsäuren selektiv zu extrahieren. Die ersten Verfahren, die hierfür zum Einsatz kamen, waren z.B. Leukozytensedimentationstechniken (Garcia A, Niubo J, Benitez MA, Viquera M, Perez JL. Comparison of two leukozyte extraction methods for zytomegalovirus antigenemia assay. J Clin Microbiol 1996, 182-184) oder Lysetechniken, bei denen z.B. die Erythrozyten zerstört werden, um diese von den Leukozyten zu trennen.

Durch Lyse kann auch die Freisetzung der RNA aus den Leukozyten durch Zugabe von Wasser und Diethylpyrocarbonate (Shi YJ, Liu, JZ. Direct reverse trancription-polymerase chain reaction from whole blood without RNA extraction. Genet Anal Tech Appl 1992, 9, 149-150) erreicht werden. Ein weiteres Beispiel für die Anreicherung oder Abreicherung von Leukozyten aus Blut ist die Verwendung von Dynabeads, die an der Oberfläche einen Anti-CD45-Antikörper tragen. Ebenso kann die Anreicherung durch mikrofluidische Verfahren mittels Margination in dünnen Kapillaren erfolgen (Shevkoplyas SS, Yoshida T. Bitensky W. Biomimetic autoseparation of Leukocytes from whole blood in microfluidic device. Anal Chem 2005, 77, 933-937; Choi J, Hyun J, Yang S. On-chip extraction of intracellular molecules in white blood cells from whole blood. Scientific Reports 2015, 5, Article Number: 15167) oder auch im Zellsorter (FACS).

Eine Alternative stellt die komplette Auflösung der zu analysierenden Zellen dar, um molekulare Komponenten wie Nukleinsäuren oder Proteine aus Serum, Zellen oder Viren anzureichern und/oder zu reinigen (Thatcher SA. DNA/RNA Preparation for Molecular Detection. Clinical Chemistry 2015. 61:189-199. Tan SC, Yiap BC. DNA, RNA, and Protein Extraction: The Past and The Present. J Biomed Biotechnol, 2009, Article ID 574398).

Die Reinigung soll dazu beitragen, dass störende Komponenten die nachfolgenden Analysen der isolierten Biomoleküle nicht negativ beeinflussen. Eine störende Komponente kann z.B. Immunglobulin G sein, was ein wesentlicher Inhibitor für die Polymerase-Kettenreaktion (PCR) aus Serum ist (Abu Al-Soud W, Jönsson LJ, Radström P. Identification and characterization fo immunglobulin G in blood as a major inhibitor of diagnostic PCR. J Clinical Microbiol 2000, 38, 345-350).

Andere Störfaktoren für molekulare Nachweise können Hämoglobin und Laktoferin aus Erythrozyten bzw. Leukozyten sein. Diese verursachen u.a. bedingt durch Eisen, welches beide Proteine enthalten, aber auch durch Abbauprodukte des Häms eine Inhibition der PCR (Abu-Al-Soud, Radström P. Purification and characterization of PCR-Inhibitory components in blood. J clin Micorbiol 2001, 39, 485-493). Der Einsatz von Nukleinsäurereinigungskits vor der Durchführung der meisten molekulardiagnostischen Tests entspricht dem heutigen Standard. Die Reagenzienkosten für diese Kits sowie Kosten für die Durchführung der Nukleinsäurereinigung sind jedoch ein beachtlicher Teil der Gesamtkosten jeder molekularen Analyse.

Der manuelle Aufwand konnte durch die Automatisierung der Nukleinsäurereinigung stark vermindert werden, doch wiegen höhere Reagenzienkosten und die Kosten für die Automatisierungstechnik diesen Vorteil teilweise wieder auf. Der direkte Einsatz von Blut in der PCR mittels Blood direct PCR Kit unter Verwendung speziell formulierter Polymerasen und Puffer (Zhang Z, Kermekchiev MB, Barnes WM. Direct DNA amplification from Crude clinical samples using PCR enhancer cocktail and novel mutants of Tac. J Mol Diagn 2010, 12, 152-161) stellt eine folgerichtige Möglichkeit dar, diese Kosten zu minimieren, hat sich jedoch für diagnostische Anwendungen bislang nicht durchgesetzt. Als Enhancer zur Verbesserung der PCR-Effizienz nutzten Zhang et al. Nonident, Trehalose und Carnitin und außerdem Omni Klentaq o. Omni Taq, was die Flexibilität von Direkt-PCR-Protokollen bei der Wahl der Reaktionsbedingungen während der Testentwicklung deutlich einschränkt und nicht für alle Inhibitoren und Targets zum Erfolg führt. Als nachteilig bei der Direct-PCR mit Blut ist auch die Schwierigkeit zu nennen, die Targetnukleinsäuren für die PCR aus den Blutzellen freizusetzen.

Im einfachsten Falle wird zur Lyse der isolierten Leukozyten das Blut eingefroren und aufgetaut, was die Nukleinsäureausbeute für die PCR erhöht. Zum Teil ist es erforderlich, präzipitiertes Blut durch Zentrifugation zu entfernen (Burkhardt J. Amplification of DNA from whole blood. PCR Methods abd Applications 1994, 3, 239-243). Werden die Erythrozyten lysiert, dann ist nach mehreren Waschschritten der verbleibenden Leukozyten ebenfalls die Real-time-PCR im Rahmen molekulargenetischer Analysen möglich (Martiniez-Serra JM, Robles J, Besalduch J. Fluorescence resonance energy transfer-based real-time polymerase chain reaction method without DNA extraction of F5, F2, F12, MTHFR and HFE. J Blood Med 2014, 5, 99-106). Ebenso können getrocknete Buffy-coat-spots direkt in der Real-time-PCR eingesetzt werden (de Gasperis MR, Caione MD, Concato C, Fiscarelli E, di Pietro N, Salotti V, Putignani L, Menichella D, Callea F. Quantitative recovery of proviral HIV-1 DNA from leukocyctes by dried buffy coat spot method for real-time PCR dtermination. J Virol Meth. 2010, 170, 121-127). Die Herstellung der Spots und das Ausstanzen von Spots aus der Matrix bedeuten jedoch für den Anwender insbesondere bei großen Probenserien einen erheblichen manuellen Aufwand.

Neben der Analyse der aus Körperflüssigkeiten wie z.B. Blut isolierten Zellen in der PCR ist es ebenso möglich, isolierte Zellen zytologisch z.B. nach Fluoreszenz-In-situ-Hybridisierung oder immunzytochemisch zu untersuchen. Es können mit diesen Methoden z.B. die Expression von Genen auf mRNA- und Proteinebene, DNA-Doppelstrangbrüche oder Mutationen untersucht werden. Eine diagnostische Anwendung stellen z.B. Lymphozytentransformationstests zur Aktivitätsmessung von Lymphozyten nach Antigenstimulation dar. In allen Fällen verbinden sich die nötigen Präparationsschritte mit einem deutlichen Zeit-, Material- und Arbeitsaufwand, der die Rentabilität der nachfolgenden Analysen beeinträchtigt.

Beispielhafte Verfahren zur Anreicherung von Nukleinsäuren des Standes der Technik sind offenbart in EP 1 167 518 A1, WO 2014/186607 A1, DE 102 36 101 A1 und DE 25 52 510 A1.

Nachteile des oben aufgeführten Standes der Technik sind zusätzliche Materialkosten für die Probenvorbereitung sowie manuell aufwändige Präparationsschritte wie z.B. die Zentrifugation im Dichtegradienten unter Einsatz von Zentrifugen oder teurer Verbrauchsmittel bei automatisierten Verfahren zur Nukleinsäurereinigung. Die gereinigten Biomoleküle müssen vor der Analyse aus den Gefäßen, in denen die Extraktion erfolgte, in die Reaktionsgefäße überführt werden, was zusätzlich eine Logistik für den Transfer und gegebenenfalls die Lagerung erforderlich macht.

Der Erfindung liegt nun die Aufgabe zugrunde, die Probleme des Stands der Technik zu vermeiden oder zumindest zu reduzieren. Insbesondere soll bei der Probenvorbereitung zur Durchführung einer PCR die Durchführung auf wenige einfache Arbeitsschritte begrenzbar sein, keine toxischen Reagenzien für z.B. die Zelllyse oder die Waschschritte verwendet werden müssen und geringe Materialkosten der bereitgestellten Reaktionsgefäße und Verbrauchsmaterialien sichergestellt sein. Die Verfahrensschritte und insbesondere das gesamte Verfahren sollen möglichst tolerant gegenüber Parameterschwankungen und äußeren Einflüssen sein, so dass der notwendige apparative Aufwand begrenzt ist. Wünschenswert ist zudem, ein möglichst einfaches Wegwaschen störender Komponenten aus der Probenmatrix in dem Maße zu ermöglichen, wie es für die jeweilige nachfolgende Analyse erforderlich ist.

Diese Aufgabe wird durch ein Verfahren mit den Merkmalen der unabhängigen Ansprüche gelöst.

Somit betrifft ein erster Aspekt der Erfindung ein Verfahren zur Anreicherung von Nukleinsäuren aus einer nukleinsäurehaltigen Probe, wobei die Probe eine dem Körper direkt entnehmbare Flüssigkeit oder Gewebe umfasst, aus einer solchen besteht oder das Produkt einer Aufbereitung einer solchen ist, wobei das Verfahren die folgenden Schritte in der angegebenen Reihenfolge umfasst:
i) Inkontaktbringen der Probe mit einer Proteinase bei einem pH-Wert von mindestens 8 und unter Ausschluss kaotroper Salze und Detergentien;
ii) Initiieren des Bindens von Nukleinsäuren der Probe an einer Oberfläche eines Probenträgers durch Einstellen eines pH-Wertes auf einen Wert kleiner 8; und anschließend
iii) Waschen des Probenträgers;
iv) Trocknen des Probenträgers mit der gebundenen Probe; und
v) Durchführen einer PCR auf demselben Probenträger, insbesondere einer quantitativen Echtzeit-Polymerase-Kettenreaktion;
wobei die Oberfläche des Probenträgers vor oder nach Schritt i) mit der Probe in Kontakt gebracht wird und wobei der Probenträger ein Polyolefin, insbesondere auszuwählen aus der Gruppe Polyethylen, Polypropylen, Polycarbonat, Polybutylen und/oder deren Copolymeren umfasst oder aus einem solchen besteht.

Überraschenderweise zeigte sich, dass die Oberfläche eines üblichen Probenträgers gegenüber den relevanten Stoffen adhäsiv ist und diese Adhäsivität durch das vorliegende Verfahren genutzt werden kann, um die Stoffe zu immobilisieren.

Darüber hinaus stellte sich heraus, dass diese Adhäsion der Moleküle beim Durchführen eines Lyseschrittes insbesondere dann in messbarer Anzahl erfolgt, wenn die Lyse mit einer Proteinase durchgeführt wird. Die Verwendung der üblicherweise verwendeten kaotropen Salze und Detergenzien umfassenden Reagenzien hingegen ist nicht erforderlich. Darunter sind insbesondere Alkohole, Gudiniumchlorid und Harnstoff in einer wirksamen Konzentration zu fassen. Unter "wirksam" ist bezogen auf Harnstoff eine höhere als die im Urin natürlich vorhandene Konzentration und bevorzugt in einer Konzentration von 4-8 M, sowie bezogen auf Tween und Triton eine Konzentration von mehr als 0,01 Gew.-% zu verstehen, da bei letztgenannten das Verfahren je 0,01 % sukzessiv messbar schlechtere Ausbeuten liefert. Der Zusatz von Detergenzien ist vorteilhaft, um Proben, die unter Standardbedingungen, wie diese in den Beispielen beschrieben wurden, schwer lysierbar sind, dennoch zu lysieren. Lysieren bedeutet die Freisetzung von Nukleinsäuren oder Nukleopeptidkomplexen in einer an den Träger bindenden Form und die simultane Bereitstellung der Bindungsbedingungen bzw. der Bindungsbedingungen nach Zugabe des Bindungsreagenzes zur lysierten Probe für die Bindung an den Träger.

Zudem sind die Produkte derartiger Reagenzien nicht PCR kompatibel und würden die Analyseergebnisse bei Konzentrationen über den genannten negativ beeinflussen. Es ist daher essentiell für die Erfindung, dass beim Durchführen einer Lyse in Schritt i) bevorzugt keine kaotropen Salze verwendet werden.

Es hat sich überraschend gezeigt, dass eine Reinigung der Probe beziehungsweise eine Isolation der Nukleinsäuren nach Verwendung des Lyseschrittes zu einer Verbesserung der Ergebnisse führen. Es wird vermutet, dass die bei der Lyse mit einer Proteinase gebildete Fragmente Komplexe mit den Nukleinsäuren (im Weiteren als Nukleinsäurepeptidkomplexe bezeichnet) eingehen, die die Adhäsion an der Probenoberfläche begünstigen. Eine weitere Erklärungsmöglichkeit ist, dass die Zellfragmente, also die Peptide, als Fängermoleküle fungieren und sozusagen Haftvermittler zwischen Oberfläche und Zielmolekül sind.

Für die Immobilisierung von Nukleinsäuren ist die Anwendung von Peptiden als Fängermoleküle besonders bevorzugt. Diese Peptide können in definierter Peptidsynthese oder durch Proteolyse aus Proteinen oder Proteingemischen hergestellt werden. Besonders effektiv in Bezug auf die Menge angereicherter Nukleinsäuren und der Verfügbarkeit sind Proteine aus der Probenmatrix nach Verdau mit einer Protease wie z.B. Proteinase K. In derart komplexen Peptidgemischen vermitteln die Peptide die Bindung zwischen der Oberfläche des Trägers und den Nukleinsäuren, so dass durch die physikochemische Vielfalt des aus dem Proteinverdau resultierenden Peptidcocktails eine Vielzahl kostengünstiger Trägermaterialien mit unterschiedlichen Oberflächeneigenschaften vergleichbarer Eignung zur Anwendung kommen können. Ähnliche Anreicherungseffekte wurden für genomische DNS unter Verwendung von Standard-PCR-Platten aus Polypropylen oder auch unter Verwendung von Nucleolink-PCR-Platten aus Polycarbonat mit Aminomodifizierung erzielt. Dadurch erreicht das Anreicherungssystem eine hohe Flexibilität in Bezug auf die Anpassung an verschiedenste Analyseverfahren. Es ist ebenso möglich, auf der Oberfläche des Trägers spezifische Fängermoleküle wie Lektine, Histone, Antikörper, Oligonukleotide oder Peptide zu immobilisieren.

Der Erfindung liegt ferner die Erkenntnis zugrunde, dass die Adhäsivität der Oberfläche des Probenträgers durch einen Bindungspuffer initiiert bzw. verbessert werden kann. Dieser Bindungspuffer wird in Form eines pH-Puffers verwendet, wobei eine Reduktion des pH-Werts der Probe die Adhäsion an der Probenträgeroberfläche verbessert.

Demnach wird die Adhäsivität der Probenträgeroberfläche durch Bildung von Nukleinsäurepeptiden aus Nukleinsäuren und Proteinfragmenten im Schritt i) und/oder die Absenkung des pH-Wertes gemäß Schritt ii) initiiert. Dabei verstärken sich die beiden Effekte gegenseitig, so dass eine Kombination beider Schritte besonders gute Immobilisierungsausbeuten bringt.

Unter adhäsiv ist vorliegend eine Oberfläche zu verstehen, welche ausgebildet ist, mit Eucyten, also kernhaltigen Zellen, Nukleoproteinkomplexen, Nukleopeptidkomplexen und/oder Nukleinsäuren eine Bindung einzugehen, so dass die Zellen oder Moleküle ausgerichtet oder willkürlich auf der Oberfläche haften und insbesondere in Waschungsprozessen nicht oder höchstens teilweise, sowohl bezogen auf die Anzahl als auch auf Teile von Zellen, von der Oberfläche heruntergespült werden. Die vorgenannte Bindung kann beispielsweise kovalent wie z.B. Schwefelbrücken sein. Alternativ handelt es sich bei der Bindung eher um koordinierende Wechselwirkungen wie beispielsweise van der Waals-Kräfte, hydrophobe Wechselwirkungen, elektrostatische Anziehung, oder Liganden-Koordinierung. Ferner ist bevorzugt, dass es sich bei den koordinierenden Wechselwirkungen um ausreichend starke elektrostatische oder polare Wechselwirkungen handelt.

Vorliegend ist unter Nukleinsäuren RNA, mRNA, miRNA, sRNA, sowie genomische DNA zu verstehen. Ferner sind zu Nukleoproteinkomplexen undegradierte Proteine in nativer und denaturierter Form mit und ohne posttranslationale Modifizierung zu verstehen, welche mit Nukleinsäuren komplexiert sind. Nukleopeptidkomplexe hingegen werden als Dipeptide bis Polypeptide, mit und ohne posttranslationale Modifizierung, wie beispielsweise Glycosylierung, Cirtrullinierung, Phosphorylierung verstanden, welche proteolytisch aus Gewebe, Serum oder Blut, aus der Autolyse von Proteasen oder aus der Peptidsynthese teilweise oder vollständig komplexiert mit DNA hergestellt sind.

DNA: z.B. genomische DNA, Plasmid-DNA, Amplifikationsprodukte, frei im Serum, Plasma oder Blut zirkulierende DNA, epigenetisch modifizierte DNA, methylierte DNA, mitochondriale DNA

In einem iii-ten Schritt wird die Oberfläche von der überstehenden Flüssigkeit getrennt und die Oberfläche des Probenträgers gewaschen, wobei erfindungsgemäß die kernhaltigen Zellen Nukleoproteinkomplexe, Nukleopeptidkomplexe und/oder Nukleinsäuren auf der Oberfläche verbleiben. An die die Schritte i bis iii umfassende Probenvorbereitung schließt sich ein Analyseschritt an, der insbesondere in flüssiger Phase auf der Oberfläche des Probenträgers stattfindet. Zur flüssigen Phase zählen erfindungsgemäß ebenfalls Moleküle, die mit Teilen des Moleküls am Träger immobilisiert sind und mit anderen Teilen in das umgebende Reaktionsmedium ragen.

Der Vorteil des erfindungsgemäßen Verfahrens im Vergleich zu üblichen ist, dass eine inhärente Adhäsivität der Probenoberfläche zur Immobilisierung spezifischer Zellen genutzt und während des Verfahrens aufrechterhalten wird. Somit entfällt die Notwendigkeit weitere Fängermoleküle zu verwenden. Zudem hat sich gezeigt, dass das erfindungsgemäße Verfahren selbst bei Verwendung von ungereinigtem Probenmaterial, also beispielsweise direkt entnommener Körperflüssigkeit, lediglich eventuell die Zugabe eines Gerinnungshemmers erforderlich macht, um signifikante und aussagekräftige Analyseergebnisse zu erhalten.

Zudem ist der Probenträger gleichzeitig das Reaktionsgefäß für die nachfolgende Analyse der Zellen oder der Bestandteile dieser Zellen. Damit ist es weder nötig, zusätzliche Gefäße für die Probenvorbereitung bereitzustellen, noch die vorbereiteten Proben in das Reaktionsgefäß zu überführen. Es ist jedoch ebenso möglich, die immobilisierten Zellen/Nukleinsäuren/Nukleinsäurenpeptidkomplexe vom Probenträger zu lösen und vor der Analyse in ein anderes Analysegefäß zu übertragen sowie insbesondere bei Teilung der Proben für mehrere Analysen diese auf mehrere Reaktionsgefäße zu verteilen. Reaktionsgefäße umfassen Standardformate in der Labordiagnostik. Diese passen zum einen in die meisten Analysegeräte für unterschiedlichste Analysen und können durch Standardliquidhandlingsysteme automatisch bearbeitet werden. Darauf baut unmittelbar ein weiterer Vorteil des erfindungsgemäßen Verfahrens auf, der darin besteht, dass durch die Bindung von Zellen aus komplexen Gemischen, wie Körperflüssigkeiten, gezielt Zellen oder zelluläre Bestandteile der Probe ausschließlich durch wenige Pipettierschritte angereichert werden können. Andere Arbeitsschritte wie Spotherstellung und Ausstanzen von Spots, Zentrifugation, Magnetseparation oder Probentransfer zwischen verschiedenen Gefäßen entfallen. Alle Arbeitsschritte, das Kontaktieren des Trägers mit der Probe, das Entfernen der Probe, Waschschritte, gegebenenfalls die Lyse von Zellen sowie das Hinzufügen der Reagenzien für die Analyse sind ausschließlich durch Pipettieren auszuführen und somit sehr leicht automatisierbar. Dies gilt insbesondere für Vollautomaten, die integriert die Probenvorbereitung als auch die nachfolgende Analyse ausführen, aber auch für kleinere Laboratorien und zur Schnellanalyse.

Bevorzugt handelt es sich bei dem Probenträger um Reaktionsgefäße wie Mikrotitrierplatte, Mikroanalyseplatten, Deckgläschen, Objektträger, Glasstäbe, Bechergläser, Kavitäten, mikrofluidische Systeme oder dergleichen mehr. Alternativ kann der Probenträger auch als Plättchen ausgeformt sein, insbesondere zusätzlich ein solches umfassen, welches weiter bevorzugt als Netz oder Sieb ausgeführt ist. Diese Ausgestaltung erhöht die funktionelle Oberfläche des Probenträgers und damit die Ausbeute, wobei gleichzeitig die Dauer des Verfahrensschritts ii) reduziert werden kann. Letzteres ist insbesondere dann der Fall, wenn der netz- oder siebartige Teil des Probenträgers durch die Flüssigkeit hindurch bewegt oder die Flüssigkeit durch den sieb- oder netzartigen Teil hindurch bewegt wird.

In bevorzugter Ausgestaltung der Erfindung ist vorgesehen, dass der Probenträger ein Polyolefin, auszuwählen aus der Gruppe Polyethylen, Polypropylen, Polybutylen und/oder deren Copolymere umfasst oder aus einem solchen besteht. Derartige Materialien zeichnen sich vorteilhafter Weise durch eine Oberfläche aus, die mit kernhaltigen Zellen, Nukleoproteinkomplexen, Nukleopeptidkomplexen und/oder Nukleinsäuren koordinative Wechselwirkungen eingeht, so dass ein diese Materialien umfassender Probenträger bereits eine Oberfläche aufweist, die zumindest zu einem geringen Maße für kernhaltigen Zellen Nukleoproteinkomplexen, Nukleopeptidkomplexen und/oder Nukleinsäuren adhäsiv wirkt, insbesondere wenn dafür geeignete Bedingungen während eines Bindungsschrittes bereitgestellt werden. Der Träger sollte gleichzeitig so ausgelegt sein, dass gebundene Analytmoleküle während des nachfolgenden Waschschrittes nicht eluiert werden. Bevorzugt jedoch durch das Reaktionsmedium der nachfolgenden Nachweisreaktion. Besonders bevorzugt ist die Beschaffenheit des Trägers in der Weise ausgelegt, dass im Bindeschritt Nukleoprotein- und / oder Nukleopeptidkomplexe binden und während eines Elutionsschrittes durch Kontakt mit einem Elutionsreagenz bzw. mit dem Nachweisreagenz nur die Nukleinsäuren eluieren und nicht die Proteine oder Peptide, was zu Nukleinsäuren höherer Reinheit führt. Umfassen jedoch die gebundenen Peptide den Analyten, können auf diese Weise alternativ störende Nukleinsäuren abgereichert werden.

Alternativ oder zusätzlich umfasst der Probenträger bevorzugt Polycarbonat, Polystyrol, Polymethacrylat, Copolymere aber auch Glas, Metall oder Keramik. Vorteilhafterweise ist das Material des Probenträgers zudem optisch transparent, semitransparent oder nicht transparent, weiß oder schwarz oder andersfarbig ausgeführt. Transparentes Material empfiehlt sich insbesondere dann, wenn die Auswertung der nachfolgenden Analyse durch den Träger hindurch, z. B. durch inverse Mikroskopie, erfolgen soll.

Eine Reduzierung einer auf die Immobilisierung beziehungsweise Adsorption bezogenen aggressiven Wirkung bzw. die Erhöhung der adsorptiven Wirkung der Oberfläche auf kernhaltige Zellen Nukleoproteinkomplexen, Nukleopeptidkomplexen und/oder Nukleinsäuren wird beispielsweise durch Anwesenheit funktioneller Gruppen der betreffenden Oberfläche erreicht. Derartige Gruppen sind insbesondere Carboxyl-, Hydroxyl-, Amino-, Sulfhydryl-, Epoxy- und Aldehydgruppen. In Abhängigkeit der konkreten Anwendung sind dem Fachmann weitere Möglichkeiten bekannt, die funktionellen Gruppen auf der Oberfläche des Trägers zu gestalten. Ebenso ist es möglich, Hydrogele auf der Oberfläche der Träger z.B. durch Polyacrylsäure oder Polyethylenglykol oder mittels Klick-Chemie aufzubringen oder durch Einbringen funktionalisierter bzw. funktionalisierbarer Vergussmassen einzubringen.

Die Vergussmassen können Einkomponenten- oder Mehrkomponenten-Vergussmassen sein. Die Aushärtung kann durch Mischen der Komponenten vor dem Einbringen bzw. durch UV-Licht oder Temperatureinwirkung nach dem Einbringen initiiert werden. Das Einbringen von Vergussmassen hat den Vorteil, dass vielfältige Materialien, aus denen die Reaktionsgefäße bestehen können, auf diese Weise einfach mit einer universellen Funktionalisierung versehen werden können. Die eingebrachten Vergussmassen können als dünner Film, als Spots oder als teilweise Verfüllung des Reaktionsraumes eingebracht werden. Als Vergussmassen werden bevorzugt Stoffe ohne störende oder mit nur geringen Eigenfluoreszenzeigenschaften wie z.B. transparente Silicone, Epoxidharze, Methacrylate oder Polystyrol verwendet. Diese können selbst funktionalisiert aber auch mit funktionalisierten Komponenten, Stoffen oder Partikeln dotiert sein.

In weiter bevorzugter Ausgestaltung der Erfindung ist vorgesehen, dass die Probe Blut, Urin, Liquor, Abstrichmaterial oder Bioptat umfasst, daraus besteht oder das Produkt einer Aufbereitung eines solchen, insbesondere als Serum oder Plasma ist. Es hat sich überraschend gezeigt, dass eine Reinigung der Körperflüssigkeit beziehungsweise eine Isolation der Nukleinsäuren insbesondere unter Verwendung des Lyseschrittes mittels detergenzienhaltigen Lyseragenzes zu einer Verschlechterung der Ergebnisse führen. Es wird vermutet, dass die bei der Lyse mit einer Proteinase gebildete Fragmente Komplexe mit den Nukleinsäuren eingehen, die die Adhäsion an der Probenoberfläche begünstigen, was durch Detergenzien vermindert wird. Andererseits können Detergenzien das Herauswaschen von Inhibitoren verbessern, so dass die Zusammensetzung des Lysereagenzes und der Waschlösung auf die Art der Probe und die geplante Analyse abzustimmen ist. Die Robustheit und Flexibilität des Verfahrens ermöglicht dies in einzigartiger und einfacher Weise.

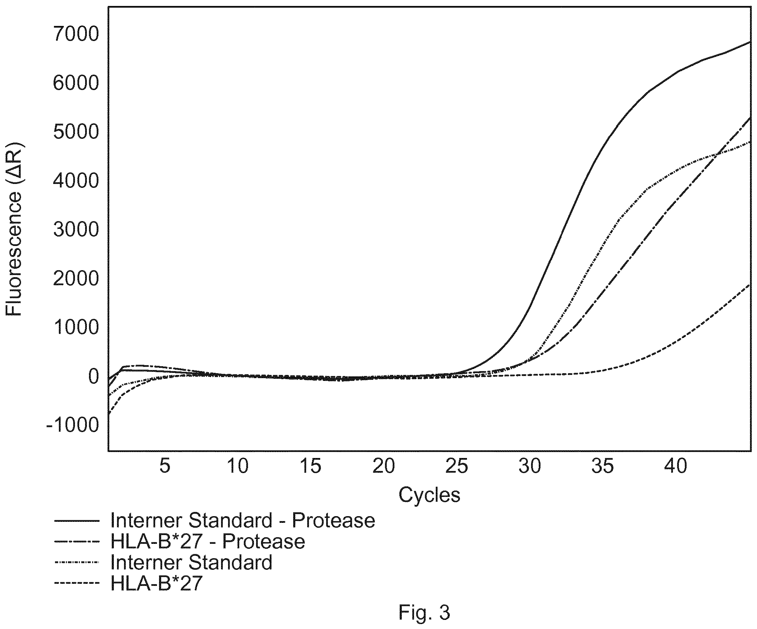

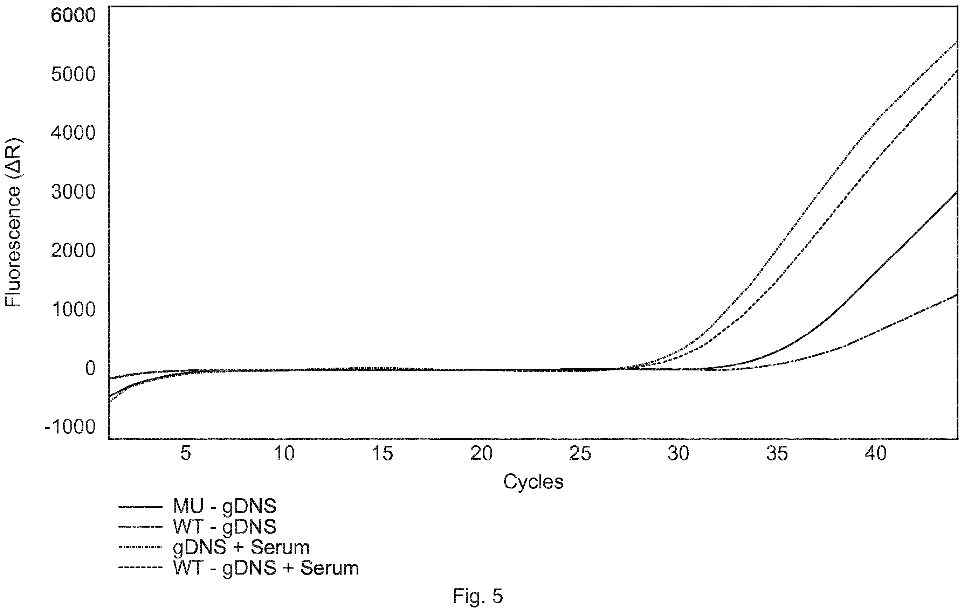

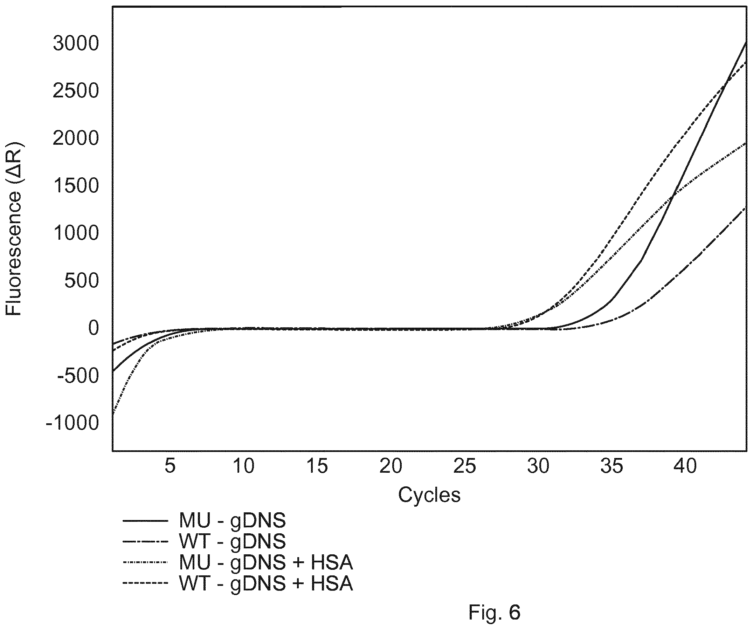

In einem besonders bevorzugten Verfahren ist vorgesehen, am Probenträger spezifisch immobilisierte Zellen oder aber Blutzellen aus mit Blut überschichteten Trägern durch Zugabe eines Lysereagenzes zu lysieren. Dadurch ist es möglich die Inhibition nachgeschalteter Nachweisreaktionen zu verringern, was sich durch weniger Ausfälle von Bestimmungen einer Probenserie zeigt bzw. durch geringere Streuungen der Messergebnisse in einer Probenserie gekennzeichnet ist. Vorteilhaft ist dabei die Anwesenheit einer Protease wie z.B. Proteinase K, Protease von Rhizopus sp. oder Protease von Aspergillus melleus im Lyseregenz mit einer Konzentration von 0,01 bis 50 U / 10 µL Probe / Lyseansatz, wenn Nukleinsäuren aus der Probe auf dem Probenträger angereichert werden sollen (Fig. 3 und 5 und 6). Besonders vorteilhaft ist Proteinase K in einer Konzentration von 0,1 U-5 U / 10 µL Probe / Lyseansatz. Durch partielle Proteolyse von Probenbestandteilen und/oder durch Zusatz von Proteinen oder Peptiden zur Probe entstehen Nukleopetid- bzw. Nukleoproteinkomplexe, die besonders effektiv in den nachfolgenden Schritten angereichert werden (Fig. 5 und 6). Somit ist bevorzugt, wenn die Proteinase aus Schritt (i) ausgewählt ist aus Proteinase K, Protease von Rhizopus sp. und/oder Protease von Aspergillus melleus im Lyseregenz vorzugsweise mit einer Konzentration im Bereich von 0,01 bis 50 U / 10 µL Probe / Lyseansatz. Diese zeigten besonders gute Immobilisierungsausbeuten.

Der pH-Wert in Schritt (i) wird auf einen Wert von mindestens 8, vorzugsweise von mindestens 9 eingestellt. Dies unterstützt die Lyse. Zur Einstellung des pH-Werts werden bevorzugt NaOH, insbeosndere 0,01-100 mM NaOH, Carbonat oder Tris und besonders bevorzugt 0,05 mM Tris-HCL verwendet, da diese auch in den Folgeschritten in der Lösung verbleiben können, ohne einen der weiterführenden Schritte zu stören. Insbesondere sind sie PCR-kompatibel.

Das Lysereagenz ist mit Vorteil hypoton, da dadurch die kernhaltigen Zellen der Probe, wie z.B. Leukozyten einfacher lysiert werden und die Puffersalze in nachfolgenden Verfahrensschritten weniger störend sind als in höherer Konzentration. Der Lyseschritt wird zwischen 10-80 °C, bevorzugt bei 25-65 °C und besonders bevorzugt zwischen 35 bis 55 °C durchgeführt. Die Dauer der Lyseschritte beträgt zwischen 1 sec bis 4 h, bevorzugt aber zwischen 1 min und 1 h, besonders bevorzugt 5 min. Durch diesen Lyseschritt kann das Verfahren auch auf andere Zellen als Eucyten wie z.B. Pilze und Bakterien bzw. auf nichtzelluläre Probenbestandteile wie z.B. Viren oder Prionen angewendet werden, die z.T. deutlich schwerer lysiert werden können als Eucyten.

Durch den Lyseschritt können ebenso zuvor auf der Oberfläche des Probenträgers immobilisierte Fängermoleküle proteolytisch abgebaut werden, so dass die Bindungskapazität der Oberfläche des Probenträgers für den nachfolgenden Bindungsschritt verbessert wird. Ein nachfolgender Bindungsschritt kann aber auch entfallen, da die Analytmoleküle bereits im Lysereagenz an den Träger binden, was für die Robustheit des Verfahrens spricht.

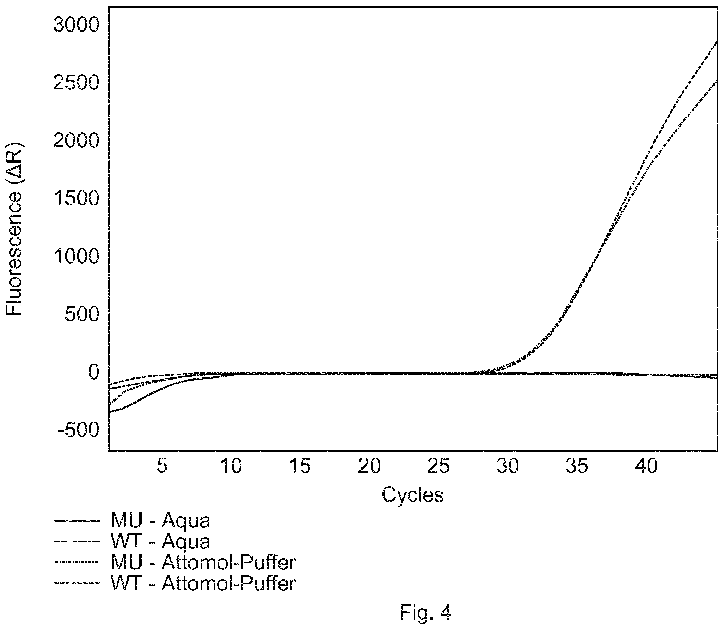

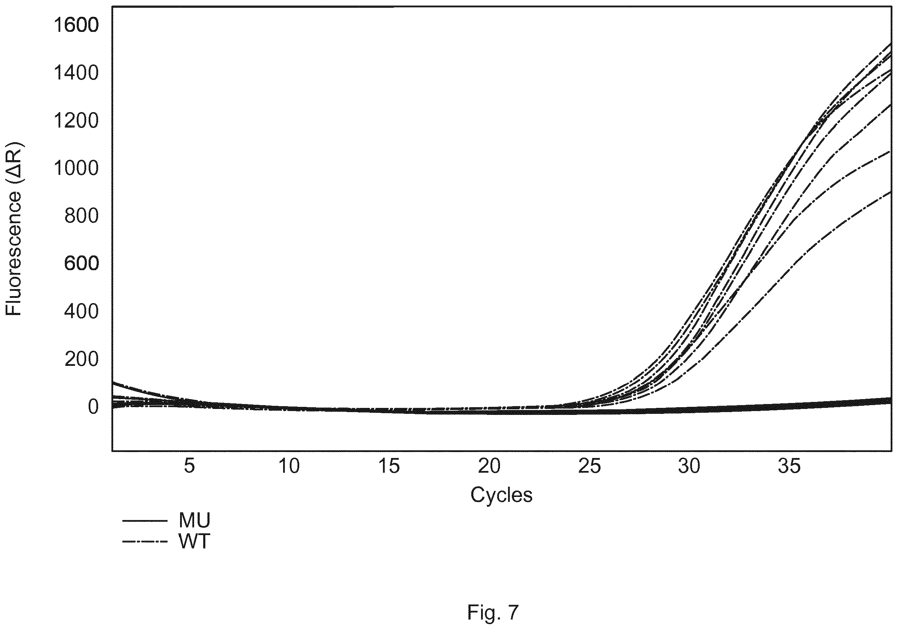

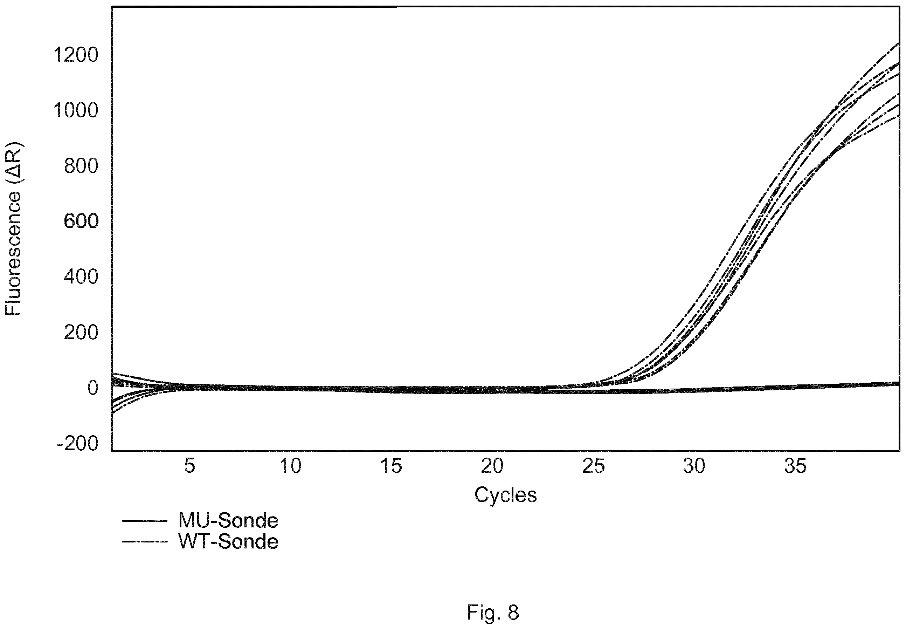

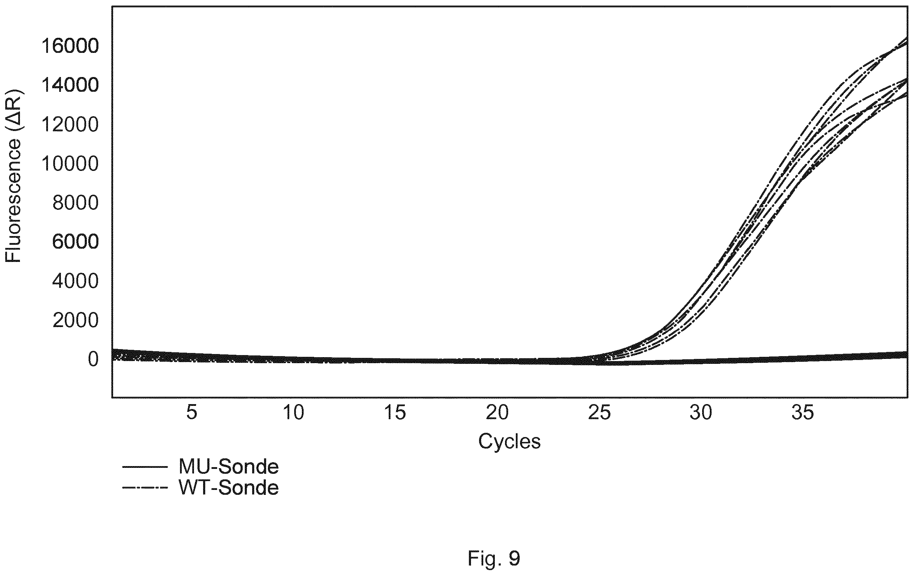

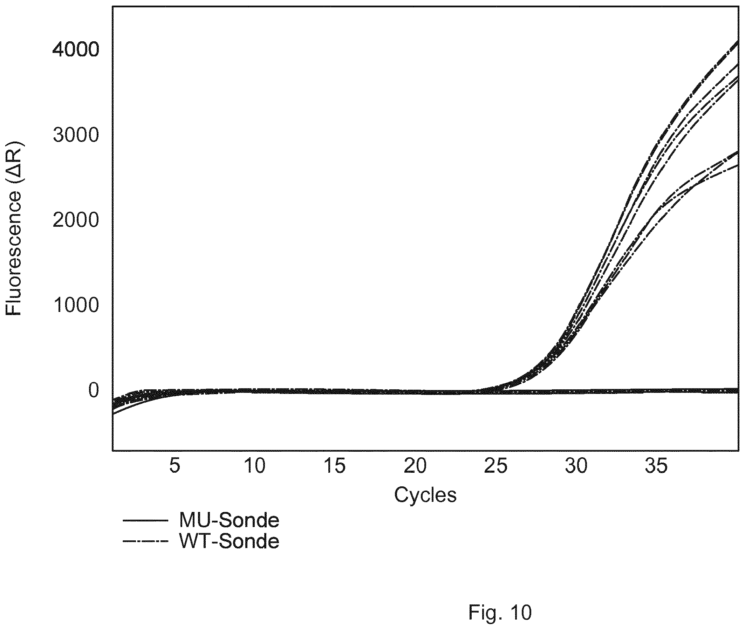

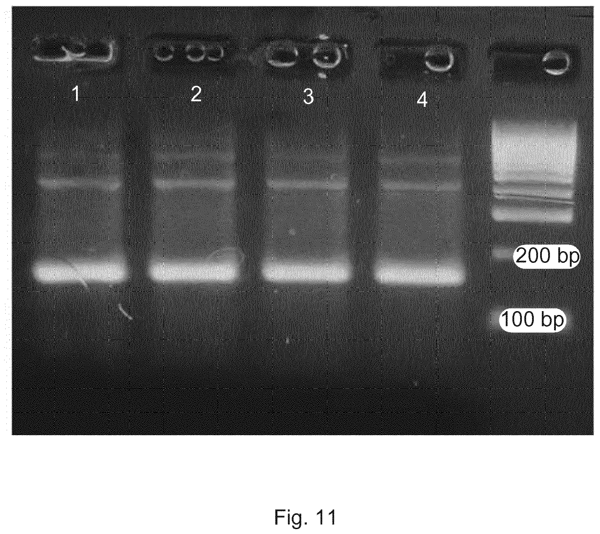

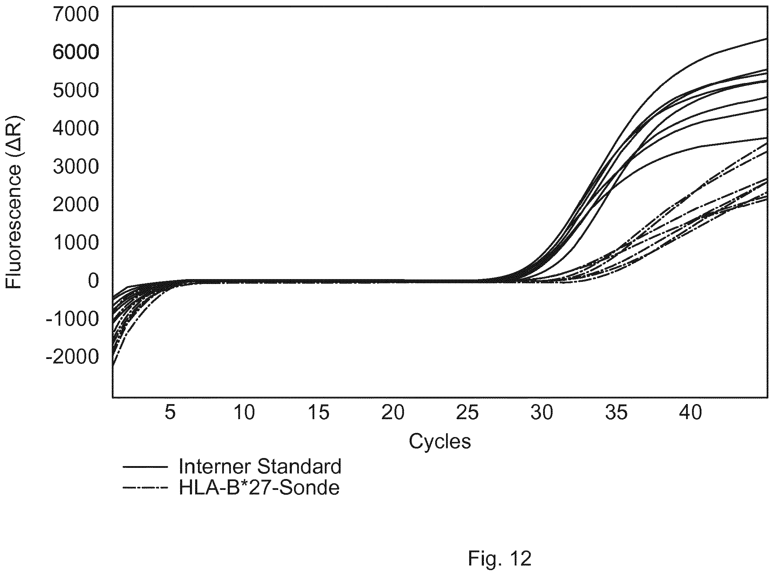

Die Bindung der in der Probe vor, während oder nach der Lyse entstanden Nukleoprotein-/Nukleopeptidkomplexe (Fig. 5 und 6) oder von Nukleinsäuren (Fig. 4) an den Probenträger kann bereits während der Lyse im Lysereagenz oder danach erfolgen. Bevorzugt wird jedoch ein Bindereagenz zum Lysereagenz gegeben, welches in der zugesetzten Menge und Konzentration den pH-Wert der Reaktionslösung in den sauren Bereich absenkt. Der pH-Wert des Gemisches liegt nach Zugabe des Bindereagenzes zwischen 1-10, bevorzugt zwischen pH 2 bis pH 5 und besonders bevorzugt bei 2,5 bis 3 und enthält bevorzugt 0,01-50 mM HCL, Citrat und/oder besonders bevorzugt Phosphat. Die Molarität des Bindungsreagenzes liegt zwischen 1-2000 mM, bevorzugt zwischen 50-800 mM und besonders bevorzugt bei 200 mM. In Abhängigkeit der Bindungseigenschaften des Probenträgers für Proteine bzw. Nukleinsäuren unter diesen Bindebedingungen binden die zu analysierenden Nukleoprotein-/Nukleopeptidkomplexe oder Nukleinsäuren an die Oberfläche des Probenträgers. Dabei ermöglicht der saure Bereich und die Anwesenheit von Phosphationen und/oder Citrationen oder anderer Ionen eine besonders hohe Beladungsdichte mit diesen Analyten, die bereits nach kurzer Bindungszeit erreicht wird (Fig. 11 und 12). Der Bindungsschritt wird zwischen 10-80 °C, bevorzugt bei 25-65 °C und besonders bevorzugt zwischen 35 bis 55 °C durchgeführt. Die Dauer des Bindeschrittes liegt bei 1 sec bis 10 h, bevorzugt zwischen 1 min und 1 h und besonders bevorzugt bei 5 min. Als Probenträger kommen bevorzugt handelsübliche Mikrotestplatten oder Reaktionsgefäße für die Durchführung von PCR oder Real-time-PCR mit hydrophober, positiv oder negativ geladener Oberfläche zur Anwendung (Fig. 7-10).

Durch Zentrifugation des Gemisches nach Zugabe des Bindungsreagenzes ist es möglich größere Aggregate von Nukleoprotein-/Nukleopeptidkomplexen oder Nukleinsäuren an die Oberfläche des Probenträgers zu sedimentieren und zu binden. Die anzuwendende Zentrifugalkraft liegt bevorzugt zwischen 1000 bis 30000 g.

Mit anderen Worten wird in bevorzugter Ausgestaltung der Erfindung in Schritt (ii) der pH-Wert durch Zugabe eines Bindungsreagenzes auf unter 6, insbesondere auf einen Wert unter 5, vorzugsweise auf einen Wert unter 3 abgesenkt. Als für das erfindungsgemäße Verfahren besonders geeignete Bindungsreagenzien erwiesen sich HCl und/oder Phosphatpuffer, Phosphat-/Citrat-Puffer, sowie Citrat-Puffer mit oder ohne Zusatz weiterer Salze wie z.B. NaCl, MgCl₂ oder CaCl₂.

Bei Durchführung des Schritts i) bedeutet das, dass der pH-Wert quasi "umgeschaltet" werden kann. Da insbesondere bei den bevorzugten Reagenzien keine störenden Nebenprodukte entstehen muss zur Analysevorbereitung lediglich ein Entfernen der überstehenden Lösung durch Abnehmen der Lösung und einen Waschschritt erfolgen. Dabei wird zum Waschen bevorzugt Wasser und insbesondere keine Detergenzien wie Alkohol oder ähnliches verwendet, die die an der Oberfläche gebundenen Nukleinsäuren und Nukleinsäurepeptidkomplexe ablösen würden.

Nach Entfernung des Lysereagenzes, des Bindungsreagenzes bzw. des Gemisches aus Lyse-und Bindereagenz vom Probenträger erfolgen keine bis mehrere, bevorzugt 1-6 und besonders bevorzugt 3 Waschschritte. Die Dauer eines Waschschrittes beträgt 0-10 min und besonders bevorzugt 0 min, d.h. der Träger wird mit der Waschlösung benetzt, die sofort wieder entfernt wird. Waschschritte werden bevorzugt mit Wasser, Isopropanol bzw. leicht sauren Puffern mit und ohne Detergenzien durchgeführt, um eine Ablösung der Analyten von der Oberfläche des Probenträgers zu vermeiden oder zu provozieren. Es kann nämlich gewünscht sein, nach dem Waschen die Analyten bevorzugt durch Zusatz eines leicht basischen Puffers möglichst effektiv abzulösen. Dazu kommen bevorzugt Tris-Lösungen oder Tris-HCL-Puffer zur Anwendung, die entsprechende Detergenzien enthalten, die die nachfolgende Nachweisreaktion nicht stören. Der Vorteil der Ablösung der Analyten vom Probenträger besteht darin, dass Analyten einer Probe auf mehrere unterschiedliche Nachweisreaktionen verteilt werden können, was die Kosten für die Präanalytik pro Probe bei Mehrfachbestimmungen stark reduzieren kann. Es ist ebenso bevorzugt, die Nachweisreaktion direkt auf dem Probenträger ohne zusätzlichen Elutionsschritt durchzuführen.

Bevorzugt wird die Oberfläche des Trägers nach Entfernung der Waschlösung bei einer Temperatur von -20 bis 100 °C, bevorzugt zwischen 70 bis 95 °C und besonders bevorzugt bei 95 °C getrocknet. Dadurch kommt es nicht zum Eintrag von Flüssigkeitsresten in den Reaktionsmix der nachfolgenden Analyse und der damit einhergehenden Konzentrationsveränderung der Mix-Bestandteile.

Das Verfahren erweist sich als unerwartet robust, da selbst unter Verwendung des Bindungsreagenzes als Lysereagenz oder umgekehrt Nukleoprotein-/Nukleopeptidkomplexe oder selbst Nukleinsäuren an den Träger binden. Dabei sinkt jedoch die Beladungseffizienz des Trägers in Abhängigkeit der Zusammensetzung der verwendeten Reagenzien und der angewendeten Oberflächeneigenschaften des Trägers.

Unter Analyseschritt ist vorliegend die Durchführung einer Polymerase-Kettenreaktion als Teil des erfindungsgemäßen Verfahrens zu verstehen. Mit besonderem Vorteil erfolgt im Anschluss an das erfindungsgemäße Verfahren eine qualitative Echtzeit Polymerase-Kettenreaktion, eine sogenannte real-time-PCR. Die erfindungsgemäße Immobilisierung von Eucyten, Nukleinsäuren und deren Komplexe aus Körperflüssigkeiten zeigte insbesondere bei diesen Folgeverfahren große Erfolge.

In weiter bevorzugter Ausgestaltung der Erfindung ist vorgesehen, dass die Polymerase-Kettenreaktion in einer Lösung auf dem Probenträger selbst durchgeführt wird. Dies hat den augenfälligen Vorteil einer Material- und Kostenersparnis sowie einen Gewinn an Ausbeute.

Besonders bevorzugt weist der Probenträger weitere Fängermoleküle auf. Je nach Verfahrensziel sind die weiteren Fängermoleküle auf die gleiche Art Zellen spezifisch oder aber auf eine andere Art. So kann zum einen die Ausbeute gleicher Zellen erhöht werden oder aber, insbesondere wenn die Fängermoleküle abschnittsweise sortiert nebeneinander angeordnet sind, gleichzeitig mehrere Zellarten immobilisiert werden.

Die Ausbeute gleicher Zellen kann weiter erhöht werden, indem der Körperflüssigkeit vor oder während Schritt ii) funktionalisierte Partikel zugesetzt werden. Dabei wird die Funktionalisierung insbesondere durch Anordnung von Fängermolekülen auf der Partikeloberfläche erreicht. Die Partikel haben bevorzugt Mikro- oder Nanogröße, weisen also bevorzugt einen Partikeldurchmesser im Bereich von 0,5-20 µm (Mikropartikel) oder kleiner (Nanopartikel) auf.

Ebenso ist es möglich, die Oberfläche der Mikropartikel so zu gestalten, dass Nukleoprotein/- peptidkomplexe oder Nukleinsäuren durch Zugabe von Lyse- und nachfolgend Bindungsreagenz in der oben für Mikrotestplatten geschilderten Weise daran binden.

Die Partikel können als Magnetpartikel, fluoreszenzkodierte, größen- oder formkodierte Partikel bereitgestellt werden, die eine Oberflächenfunktionalisierung besitzen, über die zweite Fängermoleküle gebunden sind. Zweite Fängermoleküle können den gleichen Molekülklassen wie erste Fängermoleküle entstammen, zeichnen sich durch eine gleiche aber bevorzugt durch eine andere Bindungsspezifität in Bezug auf Zellen sowie Bestandteile von Zellen oder abgegebenen Stoffen von Zellen aus.

Ferner werden die Partikel bevorzugt vor, während oder nach dem Kontaktieren der Probe mit dem Träger im Reaktionsgefäß zur Probe hinzugegeben. Diese binden in der Folge Zellen, Bestandteile von Zellen oder von Zellen abgegebene Stoffe über zweite Fängermoleküle. Durch magnetische, gravimetrische Sedimentation oder durch Flüssigkeitsströmung im Reaktionsgefäß gelangen dann die Mikropartikel in Kontakt mit dem Träger, wobei sie über die ersten Fängermoleküle direkt oder aber indirekt über die zuvor an die Partikel gebundenen Zellen, Bestandteile oder abgegebenen Stoffe an den Träger binden. Durch diese Verfahrensausgestaltung ist es möglich, Zellen, Bestandteile oder abgegebene Stoffe am Probenträger besonders stark anzureichern bzw. für die nachfolgende Analyse zugänglich zu machen. Dieses Verfahren kann z.B. eingesetzt werden, um zirkulierende Tumorzellen im Blut zu analysieren bzw. um fetale Zellen aus dem Blut der Mutter zu analysieren. Durch Verwendung von Partikeln unterschiedlich kodierter Partikelpopulationen ist es möglich, mit jeder Mikropartikelpopulation ein anderes zweites Fängermolekül einzusetzen, um Zellen, Bestandteile sowie abgegebene Stoffe von Zellen in der nachfolgenden Analyse im Multiplexformat wie z.B. bei der Erstellung von Blutbildern oder Multiplex-Antikörpernachweisen zu untersuchen.

Zusammenfassend verbleiben die Zielmoleküle also auch nach dem Waschschritt auf der Oberfläche des Probenträgers, so dass der Probenträger für den folgenden Analyseschritt verwendet werden kann und eine aufwendige Überführung in einen anderen Probenträger und damit verbundene Ausbeutereduzierung ausbleiben.

Demnach ist besonders bevorzugt, dass alle Schritte des Verfahrens die Verwendung nur eines Probenträgers umfassen, insbesondere auf demselben Probenträger eine PCR, insbesondere eine quantitative Echtzeit-Polymerase-Kettenreaktion erfolgt.

Darüber hinaus ist in Abhängigkeit der nachgelagerten Analyse der zu immobilisierenden Moleküle sowie der Art der originären Probe bevorzugt, dass das Verfahren weitere Schritte, insbesondere ein Inkubieren vor und/oder nach Schritt (ii), ein Trocknen der gebunden Probe nach Schritt (iii) und/oder ein Eluieren der gebundenen DNA, insbesondere nach Schritt (iii) umfasst. Dies kann die Ausbeute und die Analysequalität in spezifischen Fällen verbessern, ist jedoch insbesondere zur qualitativen Analyse nicht notwendig.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine fluoreszenzmikroskopische Aufnahme von immobilisierten Leukozyten nach einer bevorzugten Ausgestaltung des erfindungsgemäßen Verfahrens,
- Figur 2: eine grafische Gegenüberstellung in Form von Fluoreszenzintensitäten vs. PCR-Zyklen eines TaqMan-Mutationsnachweises nach Bindung von Zellen an einen Träger (siehe Fig. 1) aus einer erfindungsgemäßen Probe,
- Figur 3: eine grafische Gegenüberstellung in Form von Fluoreszenzintensität vs. PCR-Zyklen eines TaqMan-Mutationsnachweises in Abhängigkeit der Anwesenheit einer Protease während der Lyse einer erfindungsgemäßen Probe mit einer Probe mit Lysereagenz ohne Protease,
- Figur 4: eine grafische Gegenüberstellung in Form von Fluoreszenzintensität vs. PCR-Zyklen eines TaqMan-Mutationsnachweises in Abhängigkeit der Anwesenheit von Lysereagenz und Bindungsreagenz während der Bindung einer erfindungsgemäßen Probe mit einer Probe, die nur mit Wasser inkubiert wurde,
- Figur 5: eine grafische Gegenüberstellung in Form von Fluoreszenzintensität vs. PCR-Zyklen eines TaqMan-Mutationsnachweises in Abhängigkeit der Anwesenheit von Humanserum und humaner gDNA als erfindungsgemäße Probe mit einer Probe, die nur humane gDNS enthält,
- Figur 6: eine grafische Gegenüberstellung in Form von Fluoreszenzintensität vs. PCR-Zyklen eines TaqMan-Mutationsnachweises in Abhängigkeit der Anwesenheit von humanem Serumalbumin und humane gDNA als erfindungsgemäße Probe mit einer Probe, die nur humane gDNS enthält,
- Figur 7-10: je eine grafische Gegenüberstellung in Form von Fluoreszenzintensität vs. PCR-Zyklen eines TaqMan-Mutationsnachweises in Abhängigkeit der Anwendung verschiedener handelsüblicher Träger zur Anreicherung von Nukleinsäuren aus einer erfindungsgemäßen Probe. 7: Polypropylen transparent (Biozym, REF 712545), 8: Polypropylen frosted (Biozym, REF 712546), 9: Polypropylen weiß (Biozym, REF 712547), 10: Polypropylen weiß (Thermo Fischer, REF AB 1815100),
- Figur 11: Darstellung einer gelelektrophoretischen Auftrennung und Fluoreszenzfärbung von Amplifikationsprodukten (Lane 1-4) aus der PCR-Analyse einer erfindungsgemäßen Probe. Die Aufreinigung der Probe erfolgte unter Verwendung von Nucleolink-Riegeln, in denen anschließend auch die PCR-Analyse durchgeführt wurde,
- Figur 12-13: je eine grafische Gegenüberstellung in Form von Fluoreszenzintensität vs. PCR-Zyklen der Analyse einer erfindungsgemäßen Probe in Abhängigkeit der Dauer von Lyse- und Bindeschritt mittels TaqMan-Mutationsnachweis,
- Figur 14 -15: je eine grafische Gegenüberstellung in Form von Fluoreszenzintensität vs. PCR-Zyklen der Analyse einer erfindungsgemäßen Probe der verwendeten Inkubationsmedien beim Bindeschritt mittels TaqMan-Mutationsnachweis,
- Figur 16:: eine grafische Gegenüberstellung in Form von Fluoreszenzintensität vs. PCR-Zyklen der Analyse einer erfindungsgemäßen Probe mittels Attomol-LoopTag-Mutationsnachweis.
- Figur 17: eine grafische Gegenüberstellung in Form von Fluoreszenzintensität vs. PCR-Zyklen der Analyse einer erfindungsgemäßen Probe mittels TaqMan-Mutationsnachweis die einen großen Überschuss an humaner gDNA umfasst, zu der unterschiedliche Mengen eines Plasmids als interner Standard vor der Durchführung des erfindungsgemäßen Verfahrens gegeben wurden,
- Figur 18: eine grafische Gegenüberstellung in Form von Fluoreszenzintensität vs. PCR-Zyklen der Analyse einer erfindungsgemäßen Probe mittels TaqMan-Mutationsnachweis, wobei der Bindungsschritt mehrfach wiederholt wurde, indem das Gemisch aus Probe, Lysereagenz und Bindungsreagenz nach 5 min in ein neues Reaktionsgefäß als Träger transferiert wurde.

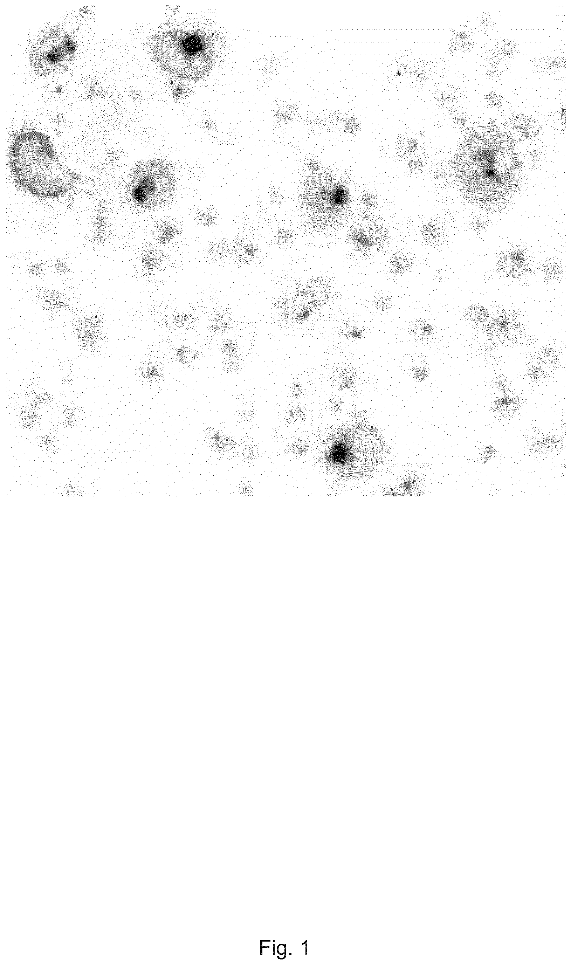

Figur 1 zeigt eine fluoreszenzmikroskopische Aufnahme von immobilisierten Leukozyten aus humanem Blut, welche durch die Fängermoleküle Histon und Phythaemagglutinin-L an einen erfindungsgemäßen Träger einer Flachbodenkavität (Mikrotestplatte) gebunden und durch Phythaemagglutinin-L-Atto647N markiert wurden. Die Auswertung erfolgte mittels Fluoreszenzmikroskop IX81 (Olympus).

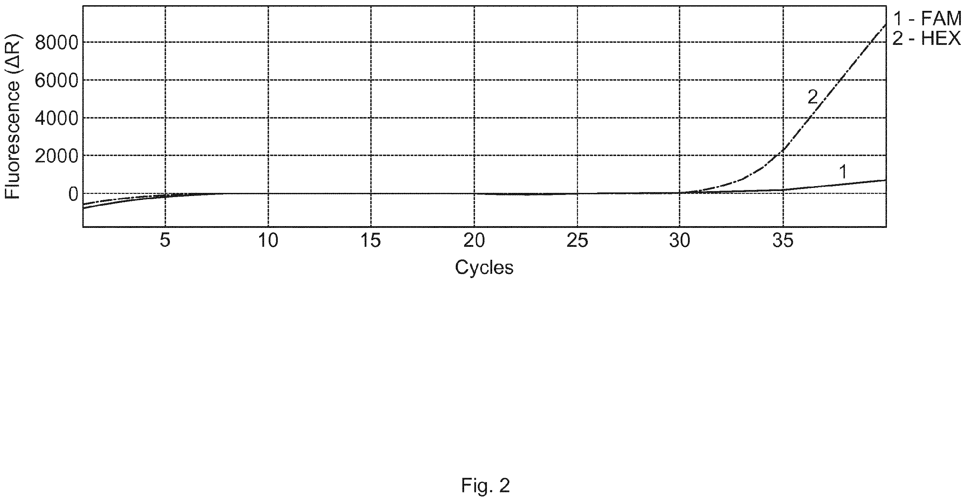

Figur 2 zeigt Zellen welche entsprechend Figur 1 in einem PCR-Tube immobilisiert und gewaschen wurden. Danach erfolgte ein Mutationsnachweis einer homozygot mutierten Probe unter Verwendung eines kommerziellen Kits (attomol-MTHFR 1298A>C Realtime TM, #1202) in Kombination mit dem Real-time-PCR-Cycler AriaMX (Agilent). 1-FAM: Signalverlauf der Wildtyp-spezifischen Sonde; 2-HEX: Signalverlauf der mutationsspezifischen Sonde.

Es zeigte sich, dass nach einer Inkubation von 0,5-20 Minuten im Falle von Blut als Probenmaterial bereits so viele Leukozyten aus dem Blut an den Träger gebunden waren, dass fast eine weitgehend geschlossene Zelllage vorliegt (Fig. 1). Durch Schütteln während der Inkubation kann die Zelldichte weiter gesteigert werden, was jedoch für die meisten Analysen dieser Zellen nicht erforderlich ist. Sind nur sehr wenige Zellen in der Körperflüssigkeit enthalten, wie z.B. bei der Isolation von zirkulierenden Tumorzellen aus Blut, wird im Abstand von bevorzugt 30 sec Intervallschütteln genutzt, um mehr Tumorzellen mit den Fängermolekülen Anti-EPCAM-Antikörper (EPCAM, Epithelial Cell Adhesion Molecule), die zuvor auf dem Träger immobilisiert wurden, in Kontakt zu bringen. Die Länge der Intervalle und die Intensität des Schüttelns richten sich nach der jeweiligen Anwendung.

Nach dem Kontaktieren der Fängermoleküle bzw. der Oberfläche des Probenträgers mit der Probe, was in Bezug auf die Art der eingesetzten Probe oder später erfolgenden Analyse unterschiedlich lange dauert und eine Lyse und einen Bindungsschritt umfassen kann, wird die Probe bevorzugt durch einen Pipettierschritt entfernt. Sollte es für die nachfolgende Analyse nötig sein, eine weitere Reduktion der Probenbestandteile vorzunehmen, werden optional Waschschritte mittels einer oder verschiedener Waschlösungen wie z.B. Wasser vorgenommen. Ein Schütteln ist meist nicht erforderlich, da durch das Pipettieren der Waschlösung allein bereits eine gute Durchmischung des Mediums erreicht werden kann. Die Waschlösung ist bevorzugt eine pH-gepufferte isotonische Waschlösung, wodurch die Zellen vor unnötiger Schädigung geschützt sind. Sollen die Zellen im Rahmen der Analyse z.B. *in vitro* kultiviert oder stimuliert werden, ist ein schonendes Waschen unerlässlich. Andererseits kann es auch das Ziel sein, die Zellen vor, während oder nach dem Waschen gezielt zu lysieren. Dies kann bevorzugt durch Zugabe einer hypotonen Lösung wie destilliertem Wasser bzw. alkalischer, saurer, alkoholischer oder hypertoner Lösungen sowie anderer Detergenzien erfolgen. Zwischen dem letzten Waschschritt und der Zugabe des Reaktionsgemisches für die Nachweisreaktion ist es von Vorteil, Flüssigkeitsreste der Waschlösung einzutrocknen bzw. aus dem Reaktionsgefäß heraus zu zentrifugieren. Dies würde die Lyse verstärken und störende Verfälschungen des Reaktionsvolumens, die letztlich zur Verdünnung oder Inhibition des Reaktionsgemisches führen können, verhindern. Besonders geeignet ist ein thermischer Lyseschritt, besonders bevorzugt nach Zugabe der Reaktionslösung für die nachfolgende Analyse z.B. mittels Real-time-PCR. Ein besonderer Vorteil der thermischen Lyse ist, dass keine Lysereagenzien in die Reaktionslösung verschleppt werden. Außerdem bleiben die Zellen und somit die natürlichen Verhältnisse, in denen die Analyten vorkommen, bis unmittelbar vor der Reaktion erhalten, was deren Kontakt mit degradierenden Enzymen reduziert. Es ist aber genauso auch möglich, Bruchstücke von Zellen oder tote Zellen zu immobilisieren, die länger gelagert oder z.B. zuvor eingefroren oder lysiert wurden. So zeigte sich das Verfahren erstaunlich robust bezüglich der Probenlagerung im Falle von Blut, in dem nach 3 Wochen Lagerung bei 4 °C oder nach Einfrieren bei -20 °C immer noch korrekte Analysen von humanen Genpolymorphismen durchgeführt werden konnten (Fig. 2). Besonders bevorzugt ist es, durch einen oder mehrere Waschschritt(e) in hypotoner Waschlösung die am Träger gebundenen Zellen zu lysieren, um Inhibitoren der Nachweisreaktion aus den Zellen zu entfernen. Hochmolekulare, nichtlösliche oder anderweitig im Zellinneren gebundene Analyten verbleiben bevorzugt in den lysierten Zellen, um die Analyten dann in der Nachweisreaktion zu detektieren. Dabei ist es durchaus vorteilhaft, die Analyten nach den Waschschritten für die Analyse aus den Zellen herauszulösen oder vom Probenträger abzulösen.

Sollen die Zellen einer mikroskopischen Analyse zugeführt werden, umfasst das Verfahren die Verwendung von Reaktionsgefäßen mit planarem, transparentem Boden wie Objektträger oder Mikrotestplatten. Um die Zellen für diesen Anwendungsfall stabilisieren zu können, werden sie bevorzugt durch Verwendung paraformaldehyd-, glutaraldehydhaltiger, oder alkoholischer Lösungen oder durch Antrocknen direkt im Reaktionsgefäß nach deren Immobilisierung auf dem Träger fixiert. Ein planarer Träger wird auch dann bevorzugt eingesetzt, wenn die sich anschließende Analyse unter Verwendung eines Biochips durchgeführt werden soll. Biochips umfassen beispielhaft in Mikrotestplatten oder auf Glasobjektträgern oder in Biosensoren gedruckte Biomoleküle als Sensormoleküle, aber auch auf dem Träger immobilisierte, fluoreszenzkodierte oder anderweitig kodierte Mikropartikel, auf deren Oberfläche populationsspezifisch Biomoleküle als Sensormoleküle gekoppelt wurden. Populationsspezifisch heißt, dass alle Mikropartikel einer Kodierung die gleichen Sensormoleküle tragen.

Figur 3zeigt eine grafische Gegenüberstellung in Form von Fluoreszenzintensität vs. PCR-Zyklen in Abhängigkeit der Anwesenheit einer Protease während der Lyse einer erfindungsgemäßen Probe mit einer Probe mit Lysereagenz ohne Protease.

Bei der Analyse komplexer Proben wie EDTA-Blut, welches sehr häufig in der molekularen Diagnostik zur Anwendung kommt, ist der Zusatz einer Protease zum Lysereagenz sehr vorteilhaft. Dadurch steigt die Sensitivität des Verfahrens, da störende Proteine abgebaut und Inhibitoren späterer Nachweisreaktionen verringert und die Analyten besser freigesetzt werden. Das äußert sich in kleineren ct-Werten und der Verringerung der Streuung der ct-Werte bzw. der Streuung und/oder der Erhöhung der Signalintensitäten bei der Real-time-PCR. Außerdem lässt sich dadurch die Zahl während der Nachweisreaktion ausgefallener Proben deutlich reduzieren. Außerdem können dadurch mehr und größere Nukleoprotein-Nukleopeptidkomplexe bereitgestellt werden, wodurch sich die Ausbeute des erfindungsgemäßen Verfahrens bezogen auf die Analytmenge erhöht. Proteine bzw. Peptide im Reaktionsansatz können Analytmoleküle vernetzen und als partikuläre oder multimolekulare Aggregate auf der Oberfläche des Trägers in besonders hoher Dichte immobilisiert werden. Ebenso werden durch die Aggregation negative Ladungen der Nukleinsäuren verringert, was deren intermolekulare Abstoßung reduziert und dadurch auch die Packungsdichte auf dem Träger unerwartet erhöht, stärker als dies durch das Bindungsreagenz allein ohne Peptide oder Proteine möglich wäre.

Figur 4 zeigt eine grafische Gegenüberstellung in Form von Fluoreszenzintensität vs. PCR-Zyklen in Abhängigkeit der Anwesenheit von Lysereagenz und Bindungsreagenz während der Bindung einer erfindungsgemäßen Probe mit einer Probe, die nur mit Wasser inkubiert wurde.

Die Robustheit des Verfahrens mit proteasehaltigem Lyse-/Bindungsreagenzgemisch äußert sich auch darin, dass sehr unterschiedlich komplexe Proben bearbeitet werden können, wenngleich die Effektivität der Anreicherung der Nukleinsäuren stark schwanken kann. Dabei ist die Anwesenheit von Serum oder auch nur von Serumalbumin oder anderen Serumproteinen wie Fibrinogen oder Immunglobulin in der Probe unerwartet von Vorteil, um mehr gDNS anzureichern als wenn die Probe ausschließlich gDNS enthält. Um wie in Fig. 3 dargestellt vergleichbare ct-Werte zu erzielen müssen deutlich größere Probenmengen reiner gDNS eingesetzt werden als im Vergleich zur Anwesenheit von Serum oder Serumproteinen im Lysereagenz.

Die Figuren 7 bis 10 zeigen je eine grafische Gegenüberstellung in Form von Fluoreszenzintensität vs. PCR-Zyklen in Abhängigkeit der Anwendung verschiedener handelsüblicher Träger zur Anreicherung von Nukleinsäuren aus einer erfindungsgemäßen Probe. 7: Polypropylen transparent (Biozym, REF 712545), 8: Polypropylen frosted (Biozym, REF 712546), 9: Polypropylen weiß (Biozym, REF 712547), 10: Polypropylen weiß (Thermo Fischer, REF AB 1815100).

In nicht erwarteter Robustheit können für das Verfahren handelsübliche Polypropylen-Mikrotestplatten als Träger eingesetzt werden, in denen Nukleinsäuren aus bevorzugt Blut angereichert und nachfolgend Analysen mittels Real-time-PCR durchgeführt werden. Während sich die Sensitivität der Analysen zwischen den verschiedenen Trägern kaum unterscheidet, was sich durch weitgehend konstante ct-Werte ausdrückt, gibt es zum Teil erhebliche Signalschwankungen bzw. Signal-/Rausch-Verhältnisse. Bezüglich der Verwendung handelsüblicher PCR-Platten ist es auch nicht zwingend notwendig, die Oberfläche dieser Träger durch Immobilisierung von Fängermolekülen oder in einer anderen geeigneten Weise zu modifizieren, was zusätzlichen Herstellungsaufwand vermeiden hilft.

Durch Fig. 11 kann gezeigt werden, dass das erfindungsgemäße Verfahren auch unter Verwendung von Trägern aus Polycarbonat, für den Fachmann unerwartet, in vergleichbarer Effizienz wie unter Verwendung von Trägern aus Polypropylen, durchgeführt werden kann.

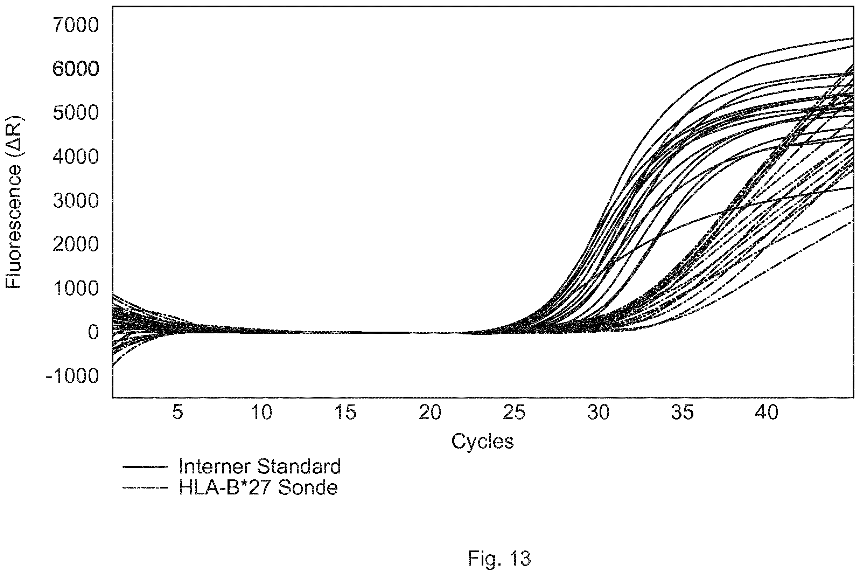

Obwohl bei Verlängerung der Zeiten für Lyse- und Bindeschritt die Sensitivität des Verfahrens steigt, was sich in geringeren ct-Werten niederschlägt, ermöglichen kürzere Inkubationszeiten eine überraschend hohe Anreicherungseffizienz von Nukleinsäuren am Träger, was bei sehr kurzer Zeit von wenigen Minuten für die Präanalytik dennoch sichere nachgeschaltete Analysen ermöglicht. Dies zeigen die Figuren 12 und 13.

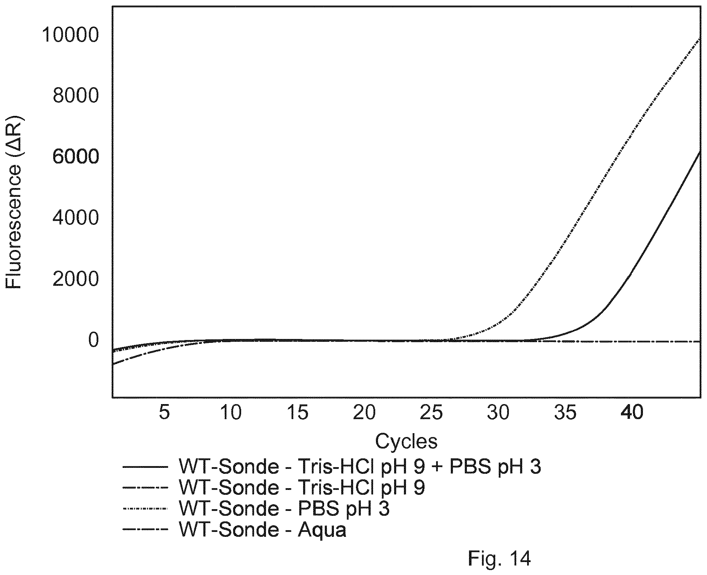

Figur 14 zeigt eine grafische Gegenüberstellung in Form von Fluoreszenzintensität vs. PCR-Zyklen der Analyse einer erfindungsgemäßen Probe der verwendeten Inkubationsmedien beim Bindeschritt.

Durch die gezielte Nutzung von Inkubationsmedien, die auch nacheinander durch Zugabe von Inkubationsmedium A zu Inkubationsmedium B zur Anwendung kommen können, ist es möglich, eine erfindungsgemäße Probe wie gDNS aus Blut oder gereinigte gDNS an der Oberfläche einer Mikrotestplatte zu immobilisieren. Andere Inkubationsmedien, wie z.B. Aqua dest. und Tris-HCL pH 9 (bei gDNS) sind dazu nicht geeignet. Tris-HCL pH 9 eignet sich nur bedingt für den Bindeschritt, sofern Blut als Probe eingesetzt wird und insbesondere zusätzlich Proteinase K enthalten ist. Für den Bindeschritt wird besonders bevorzugt PBS mit saurem pH angewendet. Insgesamt sind durch die unerwartet hohe Robustheit des Verfahrens viele Varianten möglich, aus denen die jeweils für den Anwendungszweck bevorzugte Variante selektiert und optimiert wird.

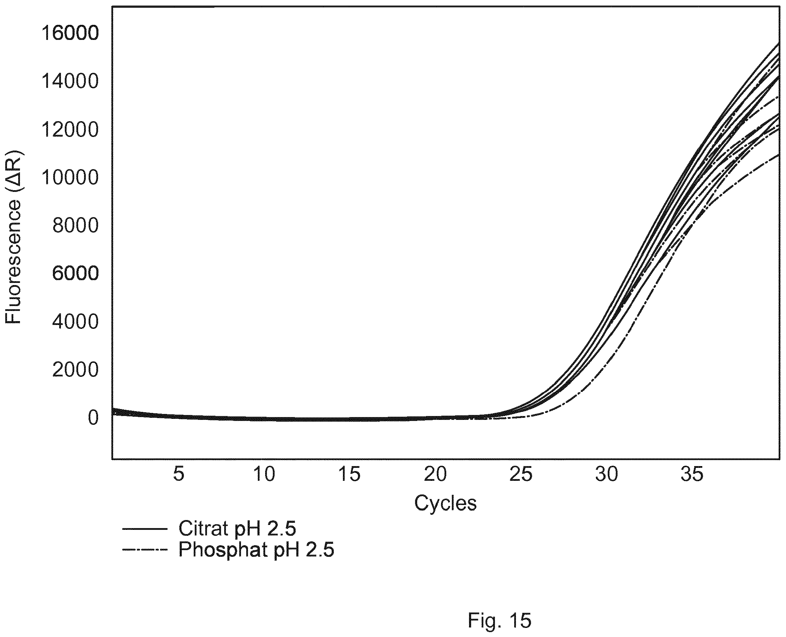

Die Flexibilität des erfindungsgemäßen Verfahrens spiegelt sich in Figur 15 wider, wonach dass Citrat-haltige Bindungsreagenz vergleichbare Ergebnisse liefert wie Phosphat-haltiges Bindereagenz.

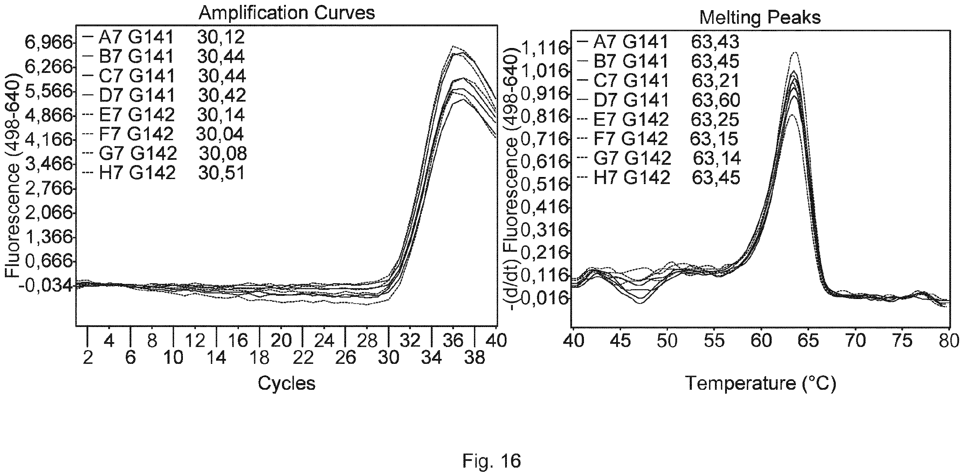

Figur 16 ist zu entnehmen, dass das erfindungsgemäße Verfahren qualitativ hochwertige Nukleinsäuren liefert, die auch mit empfindlichen Nachweisverfahren wie der Real-time-PCR und Schmelzkurvenanalyse mittels LoopTag-PCR analysiert werden können. Die Qualität der Amplifikations- und Schmelzkurven unterscheidet sich nicht von solchen Proben, die mit Standardextraktionsverfahren für Nukleinsäuren aufgearbeitet wurden.

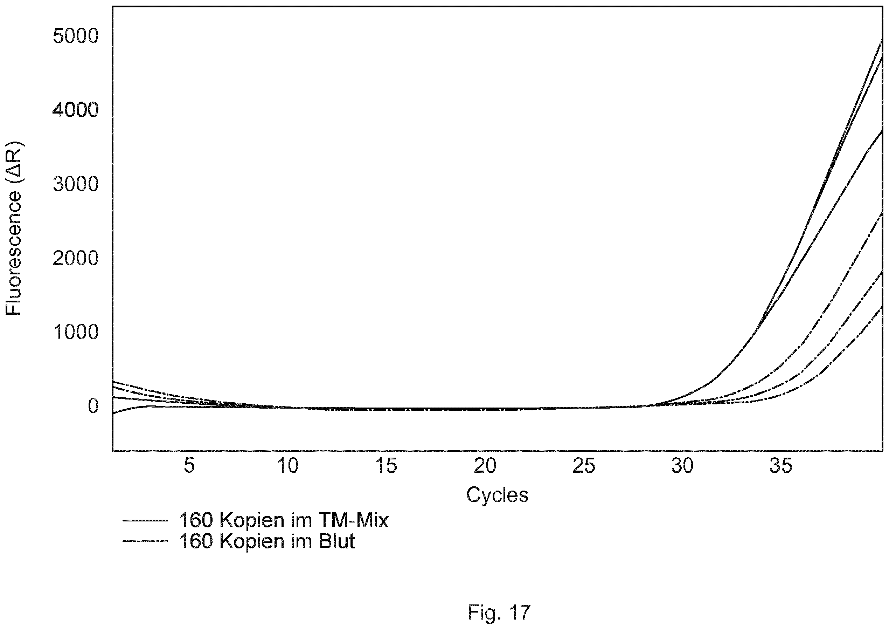

Das erfindungsgemäße Verfahren erlaubt zudem die Aufreinigung humaner gDNS aus Blut, welche mit 16, 1600 (Daten nicht gezeigt) bzw. 160 Kopien eines Plasmids (Größe ca. 5000 bp) als interner Standard versetzt wurde, wie Figur 17 zeigt. Der interne Standard kann überraschender Weise vergleichbar sensitiv nach Anreicherung mit dem erfindungsgemäßen Verfahren detektiert werden, als wenn er direkt in den PCR-Mix pipettiert wird. Der Sensitivitätsunterschied beträgt das 10-50-fache für das erfindungsgemäße Verfahren. Die Sensitivität des Verfahrens reicht aber immer noch aus, um den internen Standard im Einzelmolekülbereich zu detektieren. Dies ist besonders deshalb praxisrelevant, da viele Erreger kleine Genome oder Plasmide besitzen und in der Regel in einem großen Überschuss von gDNS in der Probe sensitiv nachgewiesen werden müssen, wie dies hier für ein Plasmid, welches als interner Aufreinigungsstandard genutzt wurde, gezeigt werden konnte. Selbst DNA-Fragmente mit einer Länge von ca. 100 bp konnten vergleichbar effizient in einem entsprechend großen Überschuss an gDNS aus Blut angereichert und detektiert werden (Daten nicht gezeigt). Damit ist das Verfahren auch für die Analyse freier gDNA in humanem Serum, die stark fragmentiert vorliegt, im Rahmen z.B. der Tumordiagnostik geeignet.

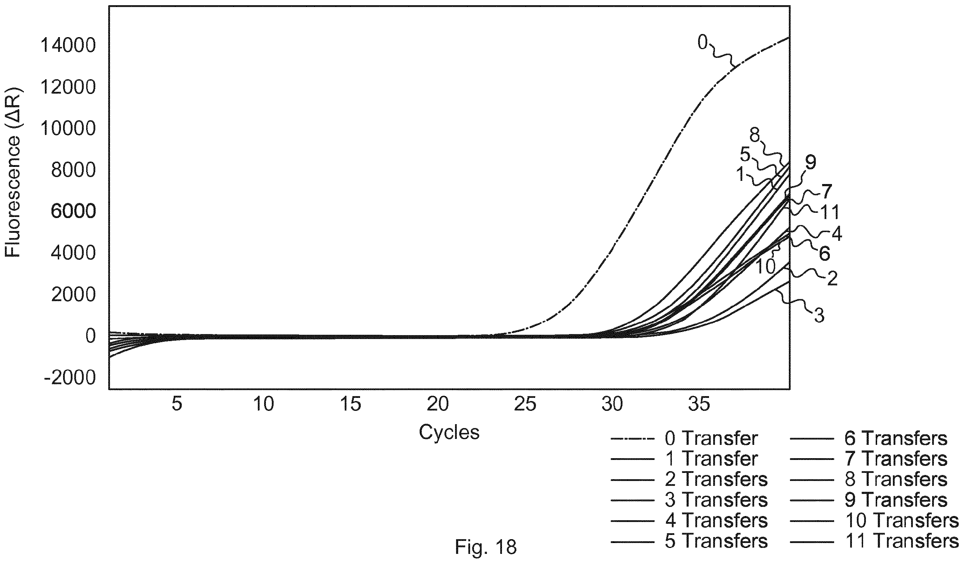

Figur 18 zeigt, dass das Verfahren den unerwarteten Vorteil aufweist, wonach bereits im ersten Bindungsschritt die größte Menge, d.h. von schätzungsweise 10-50-mal mehr des Analyten an den Träger gebunden wird als im Vergleich zu weiteren Bindungsschritten mit dem selben Gemisch aus Probe, Lyse- und Bindungsreagenz. Dadurch hält sich der resultierende Sensitivitätsverlust für viele Anwendungen, wie z.B. Erregernachweise oder Gentests in vertretbaren Grenzen.

### Ausführungsbeispiele:

### Vergleichsbeispiel 1: Lektinzytochemischer Fluoreszenznachweis

Präparation des Probenträgers:
- Bestrahlung mit UV-Licht mit einem Wellenlängenbereich von 200-400 nm
- Pipettieren von Histon- und Phythaemagglutinin-L-Lösung in Phosphatpuffer in eine Flachbodenmikrotestplatte aus Polystyrol (Greiner)
- Entfernen der Lösung aus den Kavitäten der Mikrotestplatte
- Trocknung der Mikrotestplatte bei Raumtemperatur

Immobilisierung von Leukozyten aus Blut:
- Pipettieren von Blut in die Tubes und Inkubation
- Entfernung des Blutes durch einen Pipettierschritt
- dreimaliges Waschen mit 0,1 M Natrium-Phosphatpuffer der 0,9 % NaCl enthält
- Entfernen der Waschlösung
- Zugabe und Inkubation eines Konjugates aus Phythameagglutinin-L und Atto647N
- dreimaliges Waschen mit 0,1 M Natrium-Phosphatpuffer der 0,9 % NaCl enthält
- Auswertung der Zellen im Fluoreszenzmikroskop (siehe Fig. 1) oder mittels PCR (siehe Beispiel 2).

### Beispiel 2: Real-time-PCR an thermisch lysierten Zellen

Präparation des Probenträgers:
- Pipettieren von Histon- und Phythaemagglutinin-L-Lösung in Phosphatpuffer in PCR-Tubes aus Polypropylen,
- Bestrahlung mit UV-Licht mit einem Wellenlängenbereich von 200-400 nm,
- Entfernen der Lösung aus den PCR-Tubes,
- Trocknung der PCR-Tubes bei Raumtemperatur,

Immobilisierung von Leukozyten aus Blut
- Pipettieren von Blut in die Tubes und Inkubation,
- Entfernung des Blutes durch einen Pipettierschritt,
- Zugabe eines 0,1 M Natrium-Phosphatpuffers der 0,9 % NaCl und anschließendes Waschen damit,
- Entfernen der Waschlösung,
- Zugabe des PCR-Mix entsprechend attomol-MTHFR 1298A>C Realtime TM, #1202,
- Verschließen der Tubes und Überführung in den Real-time-PCR-Cycler und Start der Real-time-PCR (siehe Fig. 2).

### Beispiel 3: Real-time-PCR an genomischer DNA (gDNA) von Blutzellen nach Lyse durch Lysereagenz und Bindung der gDNA an den Träger:

- Zugabe von 10 µL menschliches EDTA-Blut in die Kavität des Trägers
- Zugabe von 50 µL Lysegereagenz (Tris-HCl, 10 mM, pH 9) und 1 U Proteinase K,
- Inkubation 50 °C, 5 min
- Zugabe von 100 µL Bindungspuffer (PBS, 0,1 M, pH 2,5)
- Inkubation 5 min bei Raumtemperatur
- Dreimaliges Waschen mit Aqua dest.
- Entfernen der Waschlösung,
- Trocknen bei 95 °C
- Zugabe des PCR-Mix entsprechend attomol^{®} Faktor V Realtime TM (REF 1198),
- Verschließen der Tubes und Überführung in den Real-time-PCR-Cycler und Start der Real-time-PCR (siehe Fig. 15).

### Beispiel 4: Real-time-PCR an durch NaOH und Lysereagenz lysierten Zellen von Mycobacterium tuberculosis:

- Zugabe von 10 µl Sputum zum Träger + Extraktionskontrolle (siehe attomol-*Mycobacterium tuberculosis* Realtime LT2, Artikel #1190)
- Zugabe von 5 µL NaOH (5 %) und Inkubation
- Zugabe von 5 µL HCL (5 %)
- Zugabe von 50 µL Lysereagenz, Tris-HCl (5 mM, pH 9) und 1 U Proteinase K
- Inkubation, 50 °C
- Zugabe von 100 µL Bindungsreagenz, Natrium-Phosphatpuffer (0,05 M, pH 2,5)
- Inkubation
- Entfernung des Blutes durch einen Pipettierschritt,
- dreimaliges Waschen mit Aqua dest.,
- Entfernen der Waschlösung,
- Trocknen des Trägers, bei 95 °C
- Zugabe des PCR-Mix entsprechend *(attomol-Mycobacterium tuberculosis* Realtime LT2, Artikel #1190),
- Verschließen der Tubes und Überführung in den Real-time-PCR-Cycler und Start der Real-time-PCR (Daten nicht gezeigt).

Ein besonderer Vorteil des Verfahrens besteht darin, unmittelbar an den isolierten Zellen vielfältige Analysen vornehmen zu können. Dies können mikroskopische Analysen zur Bestimmung der Zytologie der Zellen sein. Es ist dabei möglich, gezielt die Expression bestimmter z.B. mRNA- oder Proteinmarker von einzelnen oder allen Zellen mittels in situ-Hybridisierung oder Immunzytochemie zu untersuchen. Es ist möglich DNA-Doppelstrangbrüche in den Leukozyten zu bestimmen, um z.B. die Strahlenbelastung des Patienten einschätzen zu können. Es ist ebenso möglich, lebende Zellen intakt am Träger zu binden und diese im Rahmen einer in vitro-Kultur oder nach einer gezielten Stimulation zu analysieren. Sehr praxisrelevante Beispiele sind Lymphozytentransformationstest oder die Bestimmung der Therapeutikaresistenz von Tumorzellen und Bakterien, die aus dem jeweiligen Probenmaterial wie z.B. Blut, Sputum oder Tumorgewebe an den Träger gebunden werden. Natürlich ist es ebenso möglich, die Zellen vor, während oder nach der Analyse bzw. der Bindung der Analyten an den Probenträger aufzulösen/abzulösen, um bestimmte Analyten für die Analyse oder weitere Analysen zugänglich zu machen. Bei solchen Analysen wie PCR, Real-time-PCR, digitale PCR werden die zu analysierenden Nukleinsäuren bevorzugt durch thermische Lyse oder Lyse mit Lysereagenz der immobilisierten Zellen unmittelbar zu Beginn der Reaktion für die Reaktion zugänglich gemacht und gleichzeitig degradierende Enzyme wie Proteasen und Nukleasen inaktiviert. Das geschieht z.B. bevorzugt während des initialen 95°C-Schrittes, bei dem Hot-Start-Polymerasen bevorzugt gleichzeitig aktiviert werden oder während des Trocknens der Platten nach Abschluss aller Waschschritte. Bei der in situ-PCR hingegen werden die Zellen durch eine chemische Fixierung stabilisiert, so dass die Analyten in den Zellen nachgewiesen werden können und die Information zur Lokalisation der Analyten erhalten bleibt. Neben diesen vielfältigen Anwendungsmöglichkeiten sind dem Fachmann natürlich weitere Ausführungsformen gemäß dem Stand der Technik bekannt. Die Erfindung umfasst die Anwendung des Verfahrens und des Kits für die medizinische und veterinärmedizinische Diagnostik und für die forensische sowie für die Life-Science-Bioanalytik.

Im Vergleich zum Stand der Technik zeichnet sich die offenbarte Erfindung demnach durch folgende Eigenschaften aus:
- Es handelt sich um ein technisch sehr einfaches Probenvorbereitungssystem, was mit nur einem Typ Arbeitsvorgang, dem Pipettieren von Analyten, d.h. Zellen, Bestandteile von Zellen oder von Zellen abgegebenen Stoffen aus der Probe in die Analyse überführt und für bestimmte Anwendungen mit einem Lyseschritt kombiniert werden kann.
- Der erfindungsgemäße Lyseschritt benötigt keine Gefahrstoffe oder hohe Konzentrationen von Salzen und Detergenzien, die eine Belastung für die Umwelt oder den Anwender darstellen.
- Durch die Zugabe von Proteinase K zur Probenlösung werden bevorzugt Proteinbruchstücke erzeugt, die im nachfolgenden Bindeschritt die Bindung des Analyten an den Träger vermitteln. Komplexe Probenmatrices sorgen dabei für einen vielfältigen Cocktail aus unterschiedlichen Peptiden, die an unterschiedlichste Trägermaterialien binden, was eine hohe Flexibilität bei der Auswahl des geeignetsten Trägers beim Testdesign führt.
- Bei Bedarf ist es mit dem Verfahren möglich, nach dem letzten Waschschritt bevorzugt mit Wasser alle die nachfolgende Analyse störenden Reagenzienrückstände zu entfernen bzw. ggf. durch Inkubation mit einem bevorzugt leicht basischen Elutionsreagenz die gebundenen Nukleinsäuren vom Träger abzulösen und auf verschiedene Analysen zu verteilen. Durch die dabei stattfindende Deprotonierung der Nukleinsäuren kommt es zu deren elektrostatischer Abstoßung von der bevorzugt hydrophoben oder geladenen Oberfläche, an der ein Großteil der Peptide gebunden bleibt. Das führt zu einer Abtrennung des Analyten von potenziell störenden Probenbestandteilen, die am Träger verbleiben.
- Probenvorbereitung und Analyse finden bevorzugt im selben Reaktionsgefäß statt, was Materialkosten und Arbeitsschritte einspart. Das gilt insbesondere für die Verwendung von Standard-Mikrotestplatten für die PCR, die nur einen Bruchteil der Kosten von z.B. Filterkartuschen für die Nukleinsäurereinigung ausmachen. Außerdem ist die Abarbeitung von größeren Probenserien auch bei manueller Arbeitsweise bei Verwendung von Mehrkanalpipetten problemlos möglich.
- Die Lyse oder Stabilisierung von Zellen zur Freisetzung oder Freilegung der Analyten, kann variabel in einer auf die jeweilige Analyse abgestimmten Weise erfolgen.
- Zytologische Analysen können mit PCR-Analysen direkt oder nacheinander kombiniert werden.
- Durch Einsatz verschiedener Fängermoleküle, oder von Mikrotestplatten mit intrinsischen Bindungseigenschaften für Analyten und von Mikropartikeln können die Analyten selektiv im Multiplexformat der Analyse zugeführt werden.
- Die thermische Lyse von Zellen während der Analyse, hilft das Ergebnis verfälschende Degradationsprozesse der Probe zu vermeiden.
- Das offenbarte Verfahren ermöglicht die Durchführung und Kombination unterschiedlichster Analyseverfahren für ein und denselben bzw. unterschiedliche Analyten der Probe.
- Ein großer Vorteil des Verfahrens besteht darin, dass auch vergleichsweise kleine Nukleinsäuren mit dem erfindungsgemäßen Verfahren angereichert werden können, selbst wenn diese sich in einem großen Überschuss von gDNS befinden. Dies ist die Voraussetzung für Erregernachweise bzw. für die Analyse von Liquid-biopsies z.B. zum Nachweis frei zirkulierender Tumor-DNA oder von Mikro-RNA.

## Patentansprüche

1. Verfahren zur Anreicherung von Nukleinsäuren aus einer nukleinsäurehaltigen Probe, wobei die Probe eine dem Körper direkt entnehmbare Flüssigkeit oder Gewebe umfasst, aus einer solchen besteht oder das Produkt einer Aufbereitung einer solchen ist, wobei das Verfahren die folgenden Schritte in der angegebenen Reihenfolge umfasst:
i) Inkontaktbringen der Probe mit einer Proteinase bei einem pH-Wert von mindestens 8 und unter Ausschluss kaotroper Salze und Detergentien;
ii) Initiieren des Bindens von Nukleinsäuren der Probe an einer Oberfläche eines Probenträgers durch Einstellen eines pH-Wertes auf einen Wert kleiner 8; und anschließend
iii) Waschen des Probenträgers;
iv) Trocknen des Probenträgers mit der gebundenen Probe; und
v) Durchführen einer PCR auf demselben Probenträger, insbesondere einer quantitativen Echtzeit-Polymerase-Kettenreaktion;
wobei die Oberfläche des Probenträgers vor oder nach Schritt i) mit der Probe in Kontakt gebracht wird;
wobei der Probenträger ein
Polyolefin, insbesondere auszuwählen aus der Gruppe Polyethylen, Polypropylen, Polycarbonat, Polybutylen und/oder deren Copolymeren umfasst oder aus einem solchen besteht.

2. Verfahren nach einem der vorhergehenden Ansprüche, **dadurch gekennzeichnet, dass** die Probe Blut, Urin, Liquor, Abstrichmaterial oder ein Bioptat umfasst, daraus besteht oder das Produkt einer Aufbereitung eines solchen, insbesondere als Serum oder Plasma, ist.

3. Verfahren nach einem der vorhergehenden Ansprüche, **dadurch gekennzeichnet, dass** die Proteinase aus Schritt (i) ausgewählt ist aus Proteinase K, Protease von Rhizopus sp. und/oder Protease von Aspergillus melleus im Lyseregenz vorzugsweise mit einer Konzentration im Bereich von 0,01 bis 50 U / 10 µL Probe / Lyseansatz.

4. Verfahren nach einem der vorhergehenden Ansprüche, **dadurch gekennzeichnet, dass** der pH-Wert in Schritt (i) auf einen Wert von mindestens 9 eingestellt wird.

5. Verfahren nach einem der vorhergehenden Ansprüche, **dadurch gekennzeichnet, dass** in Schritt (i) in der Probe vorhandene Proteine gespalten werden.

6. Verfahren nach einem der vorhergehenden Ansprüche, **dadurch gekennzeichnet, dass** in Schritt (ii) der pH-Wert durch Zugabe eines Bindungsreagenzes auf unter 6, insbesondere auf einen Wert unter 5, vorzugsweise auf einen Wert unter 3 abgesenkt wird.

7. Verfahren nach einem der vorhergehenden Ansprüche, **dadurch gekennzeichnet, dass** alle Schritte des Verfahrens die Verwendung nur eines Probenträgers umfassen.

8. Verfahren nach einem der vorhergehenden Ansprüche, **dadurch gekennzeichnet, dass** das Waschen in Schritt (iii) mittels Wasser erfolgt.

## Claims

1. Method for the enrichment of nucleic acids from a nucleic acid-containing sample, said sample comprising, consisting of, or being the product of processing a fluid or tissue directly removable from the body, said method comprising the following steps in the order indicated:
(i) contacting the sample with a proteinase at a pH of at least 8 and excluding chaotropic salts and detergents;
(ii) initiating binding of nucleic acids of the sample to a surface of a sample carrier by adjusting a pH to a value less than 8; and then
iii) washing the sample carrier;
(iv) drying the sample carrier with the bound sample; and
(v) performing PCR on the same sample carrier, in particular a quantitative real-time polymerase chain reaction;
wherein the surface of the sample carrier is brought into contact with the sample before or after step i);
where the sample carrier comprises or consists of a
polyolefin, in particular selectable from the group of polyethylene, polypropylene, polycarbonate, polybutylene and/or copolymers thereof.

2. Method according to any one of the preceding claims, **characterized in that** the sample comprises, consists of or is the product of processing blood, urine, cerebrospinal fluid, smear material or a bioptate, in particular as serum or plasma.

3. Method according to any one of the preceding claims, **characterized in that** the proteinase of step (i) is selected from proteinase K, protease from Rhizopus sp. and/or protease from Aspergillus melleus in the lysis reagent preferably with a concentration in the range of 0.01 to 50 U / 10 µL sample / lysis reagent.

4. Method according to any one of the preceding claims, **characterized in that** the pH is adjusted to a value of at least 9 in step (i).

5. Method according to any one of the preceding claims, **characterized in that** proteins present in the sample are cleaved in step (i).

6. Method according to one of the preceding claims, **characterized in that** in step (ii) the pH is lowered to below 6, in particular to a value below 5, preferably to a value below 3, by addition of a binding reagent.

7. Method according to any of the preceding claims, **characterized in that** all steps of the method comprise the use of only one sample carrier.

8. Method according to any one of the preceding claims, **characterized in that** the washing in step (iii) is carried out by means of water.

## Revendications

1. Procédé d'enrichissement des acides nucléiques d'un échantillon contenant des acides nucléiques, ledit échantillon comprenant un liquide ou un tissu pouvant être prélevé directement sur le corps, consistant en un tel liquide ou tissu ou étant le produit d'une préparation d'un tel liquide ou tissu, le procédé comprenant les étapes suivantes dans l'ordre indiqué :
i) mise en contact de l'échantillon avec une protéinase à un pH d'au moins 8 et en l'absence de sels et de détergents chaotropiques ;
ii) initiation de la liaison des acides nucléiques de l'échantillon à une surface d'un porte-échantillon en ajustant le pH à une valeur inférieure à 8 ; et ensuite
iii) le lavage du porte-échantillon ;
iv) séchage du porte-échantillon avec l'échantillon lié ; et
v) réalisation d'une PCR sur le même porte-échantillon, en particulier d'une réaction en chaîne par polymérase quantitative en temps réel ;
la surface du porte-échantillon étant mise en contact avec l'échantillon avant ou après l'étape i) ;
le porte-échantillon étant un
polyoléfine, en particulier choisie dans le groupe constitué par le polyéthylène, le polypropylène, le polycarbonate, le polybutylène et/ou leurs copolymères, ou est constituée d'une telle polyoléfine.

2. Procédé selon l'une des revendications précédentes, **caractérisé en ce que** l'échantillon comprend du sang, de l'urine, du liquide cérébrospinal, du matériel de frottis ou un bioptate, en est constitué ou est le produit d'une préparation de celui-ci, en particulier sous forme de sérum ou de plasma.

3. Procédé selon l'une quelconque des revendications précédentes, **caractérisé en ce que** la protéinase de l'étape (i) est choisie parmi la protéinase K, la protéase de Rhizopus sp. et/ou la protéase d'Aspergillus melleus dans le réactif de lyse, de préférence avec une concentration dans la plage de 0,01 à 50 U / 10 µL d'échantillon / masse de lyse.

4. Procédé selon l'une quelconque des revendications précédentes, **caractérisé en ce que** le pH est ajusté à une valeur d'au moins 9 à l'étape (i).

5. Procédé selon l'une quelconque des revendications précédentes, **caractérisé en ce que**, à l'étape (i), des protéines présentes dans l'échantillon sont clivées.

6. Procédé selon l'une quelconque des revendications précédentes, **caractérisé en ce que**, à l'étape (ii), le pH est abaissé à une valeur inférieure à 6, notamment à une valeur inférieure à 5, de préférence à une valeur inférieure à 3, par addition d'un réactif de liaison.

7. Procédé selon l'une quelconque des revendications précédentes, **caractérisé en ce que** toutes les étapes du procédé comprennent l'utilisation d'un seul porte-échantillon.

8. Procédé selon l'une quelconque des revendications précédentes, **caractérisé en ce que** le lavage à l'étape (iii) est effectué au moyen d'eau.
